(19)

**Europäisches Patentamt**
**European Patent Office**
**Office européen des brevets**

(11) **EP 4 342 436 B1**

(12) **EUROPÄISCHE PATENTSCHRIFT**

(45) Veröffentlichungstag und Bekanntmachung des Hinweises auf die Patenterteilung:
**15.01.2025  Patentblatt 2025/03**

(21) Anmeldenummer: **24150966.0**

(22) Anmeldetag: **12.11.2007**

(51) Internationale Patentklassifikation (IPC):
**A61F 9/01** *(2006.01)*     **A61F 9/008** *(2006.01)*
**A61F 9/009** *(2006.01)*

(52) Gemeinsame Patentklassifikation (CPC):
**A61F 9/008; A61F 9/00827;** A61F 9/00829;
A61F 9/00836; A61F 9/00838; A61F 9/009;
A61F 2009/00872; A61F 2009/00897

(54) **LASERBASIERTE BEHANDLUNGSVORRICHTUNG ZUR OPERATIVEN FEHLSICHTIGKEITSKORREKTUR**

LASER-BASED TREATMENT DEVICE FOR SURGICAL CORRECTION OF POOR EYESIGHT

DISPOSITIF DE TRAITEMENT LASER DESTINÉ À LA CORRECTION OPÉRATIONNELLE DES TROUBLES DE LA VUE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität:  **10.11.2006   DE 102006053118**
         **10.11.2006   US 85820106 P**

(43) Veröffentlichungstag der Anmeldung:
**27.03.2024   Patentblatt 2024/13**

(62) Dokumentnummer(n) der früheren Anmeldung(en) nach Art. 76 EPÜ:
**20215994.3 / 3 831 348**
**15200150.9 / 3 037 077**
**10173881.3 / 2 298 255**
**07846562.2 / 2 086 482**

(73) Patentinhaber: **Carl Zeiss Meditec AG**
**07745 Jena (DE)**

(72) Erfinder:
• **BISCHOFF, Mark**
  **07749 Jena (DE)**
• **STICKER, Markus**
  **07749 Jena (DE)**
• **STOBRAWA, Gregor**
  **07743 Jena (DE)**
• **BERGT, Michael**
  **99425 Weimar (DE)**
• **WIECHMANN, Martin**
  **07743 Jena (DE)**

(74) Vertreter: **Patentanwälte Geyer, Fehners & Partner mbB**
**Perhamerstrasse 31**
**80687 München (DE)**

(56) Entgegenhaltungen:
**EP-A1- 1 153 584     EP-A1- 1 719 483**
**DE-A1- 10 334 109     US-A- 6 099 522**
**US-A1- 2003 069 566     US-A1- 2004 243 112**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Eine Planungseinrichtung zum Bestimmen von Steuerdaten für eine Behandlungsvorrichtung zur operativen Fehlsichtigkeitskorrektur eines Auges eines Patienten wird beschrieben, wobei die Planungseinrichtung die Steuerdaten erzeugt für ein Behandlungsvorrichtung, die aufweist eine Lasereinrichtung, welche durch Einstrahlen gepulster Laserstrahlung Hornhaut-Gewebe trennt, wobei die Laserstrahlung auf in einem Muster in der Hornhaut liegende Zielpunkte fokussiert ist.

Die Erfindung bezieht sich auf eine Behandlungsvorrichtung gemäß der Ansprüche zur operativen Fehlsichtigkeitskorrektur eines Auges eines Patienten, die aufweist eine Schnittstelle zum Zuführen von Messdaten über Parameter des Auges und Fehlsichtigkeitsdaten über die zu korrigierenden Fehlsichtigkeit des Auges, eine Lasereinrichtung, die durch Einstrahlen gepulster Laserstrahlung Hornhaut-Gewebe trennt, wobei die Laserstrahlung auf in einem Muster in der Hornhaut liegende Zielpunkte fokussiert ist.

Weiterhin wird ein Verfahren zur Vorbereitung von Steuerdaten für eine Behandlungsvorrichtung zur operativen Fehlsichtigkeitskorrektur eines Auges eines Patienten beschrieben, welches nicht Teil des beanspruchten Gegenstands der Erfindung ist, die eine Lasereinrichtung aufweist, welche durch Einstrahlen gepulster Laserstrahlung Hornhaut-Gewebe trennt, wobei die Lasereinrichtung im Betrieb die Laserstrahlung gemäß den Steuerdaten auf in einem Muster in der Hornhaut liegende Zielpunkte fokussiert.

Der klassische Weg um Fehlsichtigkeit des menschlichen Auges zu korrigieren, ist seit langer Zeit die Brille. Mittlerweile wird jedoch zunehmend auf refraktive Chirurgie zurückgegriffen, die durch Veränderungen der Augenhornhaut eine Fehlsichtigkeitskorrektur bewirkt. Ziel aller Operationsmethoden ist es dabei, die Hornhaut gezielt zu verändern, um so die Lichtbrechung zu beeinflussen. Hierfür sind unterschiedliche Operationsmethoden bekannt. Die EP 1719483 A1 befasst sich mit der Erzeugung von Daten für die lasergestützte Fehlsichtigkeitskorrektur. Am weitverbreitetsten ist die sogenannte Laser-Insitu-Keratomileusis, die auch LASIK abgekürzt wird. Dabei wird zuerst eine Hornhautlamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Laserkeratoms, wie es z.B. Intralase Corp., Irvine, USA vertreibt.

Letzteres erzeugt in der Hornhaut durch Laserstrahlung eine Schnittfläche. Dabei laufen im Gewebe zeitlich hintereinander mehrere Prozesse ab, die durch die Laserstrahlung initiiert werden. Liegt die Leistungsdichte der Strahlung über einen Schwellwert, kommt es zu einem optischen Durchbruch, der in der Hornhaut eine Plasmablase erzeugt. Die Plasmablase wächst nach Entstehen des optischen Durchbruches durch sich ausdehnende Gase. Wird der optische Durchbruch nicht aufrecht erhalten, so wird das in der Plasmablase erzeugte Gas vom umliegenden Material aufgenommen und die Blase verschwindet wieder. Es sind auch gewerbetrennende Effekte möglich, die ohne Plasmablase wirken. Der Einfachheit halber werden all solche Prozesse hier unter dem Begriff "optischer Durchbruch" zusammengefasst, d.h. dieser Begriff soll nicht nur den optischen Durchbruch sondern auch die daraus resultierenden Wirkungen in der Hornhaut einschließen.

Zur Erzeugung des optischen Durchbruches wird die Laserstrahlung gepulst angewendet, wobei die Pulslänge unter 1 ps liegt. Dadurch wird die zur Auslösung eines optischen Durchbruches nötige Leistungsdichte für den jeweiligen Puls nur in einem engen räumlichen Gebiet erreicht. Die US 5984916 zeigt diesbezüglich deutlich, dass der räumliche Bereich des optischen Durchbruches (in diesem Fall der erzeugten Wechselwirkung) stark von der Pulsdauer abhängt. Eine hohe Fokussierung des Laserstrahls in Kombination mit den erwähnten kurzen Pulsen erlaubt es damit, den optischen Durchbruch punktgenau in der Hornhaut einzusetzen.

Zur Erzeugung der dünnen Lamelle wird nun mit dem Laserkeratom eine Serie optischer Durchbrüche an vorbestimmten Stellen so erzeugt, dass eine Schnittfläche ausgebildet wird, welche die Lamelle von der darunterliegenden Hornhaut löst.

Nachdem die Lamelle gelöst und zur Seite geklappt wurde, ist bei der LASIK-Operation die Anwendung eines Excimer-Lasers vorgesehen, der das nun freiliegende Hornhautgewebe durch Ablation abträgt. Nachdem auf diese Art und Weise in der Hornhaut liegendes Volumen verdampft wurde, wird die Hornhautlamelle wieder auf den ursprünglichen Platz zurückgeklappt. Die bereits in der Anwendung befindliche LASIK-Methode, die, soweit ein Laserkeratom zum Einsatz kommt, als auch als fs-LASIK bezeichnet wird, legt somit eine deckelförmige Hornhautlamelle frei, klappt diese ab und ablatiert das freigelegte Gewebe mit einem Ablationslaser.

Es ist im Stand der Technik auch erwähnt, dass die Fehlsichtigkeitskorrektur dadurch erzeugt wird, dass mittels der gepulsten Laserstrahlung ein linsenförmiges Teilvolumen im Hornhautgewebe isoliert wird. Eine entsprechende Schilderung findet sich beispielsweise in der WO 2005/011545 A1. Entsprechende Geräte sind jedoch am Markt noch nicht verfügbar.

Bei der LASIK-Methode wird die Ablation des mittels Keratom freigelegten Hornhautgewebes durch einen Ablationslaser so durchgeführt, dass damit ein gewünschtes Volumen abgetragen wird. Der Laserstrahl wird dazu an verschiedene Stellen auf die freigelegte Hornhaut fokussiert, um das Material abzutragen. Der Materialabtrag in der Hornhaut wird durch ein sogenanntes shot file eingestellt, das die Anzahl der Ablationslaserstrahlungspulse und deren jeweilige Koordinaten, auf die die Pulse abgegeben werden, festlegt. Die Erzeugung des shot file erfolgt in den Geräten nach vorheriger Vermessung des Auges. Es ist für die derzeit wissenschaftlich untersuchten Operationsverfahren und -geräte, bei denen ein Volumen in der Hornhaut isoliert wird, aufgrund des andersartigen Arbeitsprinzips nicht brauchbar.

Der Erfindung liegt deshalb die Aufgabe zu Grunde, ein System zur Fehlsichtigkeitskorrektur anzugeben, so dass eine operative Fehlsichtigkeitskorrektur einfach erfolgen kann.

Die Aufgabe wird gemäß dem unabhängigen Anspruch gelöst.

Eine Planungseinrichtung ist so ausgebildet, dass sie aus zugeführten Mess- und Fehlsichtigkeitsdaten ein Volumen definiert, das innerhalb der Hornhaut liegt und dessen Entfernung aus der Hornhaut die gewünschte Fehlsichtigkeitskorrektur bewirkt, eine Grenzfläche festlegt, die das definierte Volumen innerhalb der Hornhaut begrenzt, und für diese Grenzfläche einen Steuerdatensatz zur Ansteuerung der Lasereinrichtung erzeugt, der in der Hornhaut ein dreidimensionales Muster der Zielpunkte festlegt, die in der Grenzfläche liegen und so angeordnet sind, dass die Grenzfläche nach Einstrahlung der gepulsten Laserstrahlung gemäß des Steuerdatensatzes als Schnittfläche ausgebildet ist, die das definierte Volumen in der Hornhaut isoliert und so entfernbar macht.

Eine Behandlungsvorrichtung zur operativen Fehlsichtigkeitskorrektur eines Auges eines Patienten weist auf eine Schnittstelle zum Zuführen von Messdaten über Parameter des Auges und Fehlsichtigkeitsdaten über die zu korrigierenden Fehlsichtigkeit des Auges, eine Lasereinrichtung, welche durch Einstrahlen gepulster Laserstrahlung Hornhaut-Gewebe trennt, wobei die Laserstrahlung auf in einem Muster in der Hornhaut liegende Zielpunkte fokussiert ist, und eine Planungseinrichtung der im vorigen Absatz beschriebenen Art.

Ein Verfahren der eingangs genannten Art weist folgende Schritte auf: Ermitteln von Messdaten über Parameter des Auges und Fehlsichtigkeitsdaten über die zu korrigierenden Fehlsichtigkeit des Auges, Definieren eines Volumen aus den Messdaten und den Fehlsichtigkeitsdaten, wobei das Volumen innerhalb der Hornhaut liegt und wobei die nach Betrieb der Behandlungsvorrichtung vorgesehene Entfernung des Volumens aus der Hornhaut die gewünschte Fehlsichtigkeitskorrektur zur Folge hat, Festlegen einer Grenzfläche, die das definierte Volumen innerhalb der Hornhaut begrenzt, und Festlegen eines dreidimensionales Muster von Zielpunkten in der Hornhaut, wobei die Zielpunkte in der Grenzfläche liegen und so angeordnet sind, dass die Grenzfläche bei Einstrahlung der gepulsten Laserstrahlung gemäß der Steuerdaten als Schnittfläche ausgebildet wird, die das definierte Volumen in der Hornhaut isoliert und so entfernbar macht, Erzeugen eines Steuerdatensatzes enthaltend das dreidimensionale Muster zur Ansteuerung der Lasereinrichtung.

Es kann ausgehend von Mess- und Fehlsichtigkeitsdaten des zu korrigierenden Auges zuerst ein Volumen definiert werden, das innerhalb der Hornhaut liegt und dessen Entfernung die gewünschte Fehlsichtigkeitskorrektur bewirkt. Die Entfernung kann z.B. über einen das Volumen zugänglich machenden Schnitt zur Hornhautoberfläche erfolgen, dessen Erzeugung ebenfalls von der Planungseinrichtung bzw. den Steuerdaten bewirkt wird. Damit die Korrektur bei Tag- wie Nachtsehen möglichst gleich ist, sollte das Volumen möglichst die Pupille des dunkelangepassten Auges überdecken. Für dieses Volumen wird dann mindestens eine Grenzfläche festgelegt, bevorzugt mehrere, die das Volumen begrenzen. Die Grenzfläche(n) wird/werden mit der Behandlungsvorrichtung bzw. deren Lasereinrichtung später als Schnittflächen ausgebildet, um das Volumen entfernen, beispielsweise entnehmen zu können. Für die Grenzfläche(n) wird ein Steuerdatensatz ermittelt, der in der Grenzfläche liegende Zielpunkte vorgibt, an denen jeweils ein optischer Durchbruch mittels der Laserstrahlung zu erzeugen ist, um die Schnittfläche zu bilden. Die Zielpunkte sind ein dreidimensionales Muster und liegen sämtlich in der zuvor definierten Grenzfläche. Die Planungseinrichtung kann dabei auch Bestandteil der Lasereinrichtung sein.

Als Ergebnis der Verfahrens bzw. der Tätigkeit der Planungseinrichtung liegt ein Steuerdatensatz vor, der eine automatische Ansteuerung der Lasereinrichtung ermöglicht, so dass die Erzeugung der Schnittflächen, welche das Volumen in der Hornhaut isolieren, dann automatisch ablaufen kann. Die Planungseinrichtung bzw. die damit ausgerüstete Behandlungsvorrichtung zur operativen Fehlsichtigkeitskorrektur sowie das Verfahren zum Bereitstellen von Steuerdaten für eine Behandlungsvorrichtung erzeugt vorzugsweise den Steuerdatensatz automatisch aus den bereitgestellten Messdaten und Fehlsichtigkeitsdaten. Eine Mitwirkung des Operateurs ist dabei in vorteilhaften Ausgestaltungen nicht erforderlich.

Die Planungseinrichtung kann als Computer ausgebildet sein, der durch ein Programm gesteuert arbeitet. Optional kann die Planungseinrichtung Bestandteil der Behandlungsvorrichtung sein. Ein entsprechendes Computerprogrammprodukt mit Programmcode, der die erwähnten Verfahrensschritte bewirkt, ist damit ebenfalls eine Lösung der erwähnten Aufgabe.

Die Erzeugung des Steuerdatensatzes basiert auf ermittelten Messdaten und Fehlsichtigkeitsdaten des Auges. Diese Messdaten können an einer eigenständigen Messeinrichtung erfasst werden. Zweckmäßigerweise ist jedoch die Behandlungsvorrichtung direkt mit der Messeinrichtung verbunden. Als Messeinrichtung kommen beispielsweise ein Autorefraktor, ein Refraktometer, ein Keratometer, eine Aberrometer, ein OCT oder eine Wellenfrontvermessungseinrichtung oder eine beliebige Kombination aus solchen Einrichtungen bzw. Messgeräten in Frage.

Die Fehlsichtigkeit kann Hyperopie, Myopie, Presbyopie, Astigmatismus, gemischten Astigmatismus (Astigmatismus, bei dem in einer Richtung Hyperopie und in einer rechtwinklig dazu liegenden Richtung Myopie vorliegt), asphärische Fehler und Aberrationen höherer Ordnung umfassen.

Eine direkte Verbindung der Messeinrichtung mit der Planungseinrichtung oder der damit ausgerüsteten Behandlungsvorrichtung hinsichtlich der Datenübertragung, was in einer Variante verwendet werden kann, hat den Vorteil, dass die Verwendung falscher Mess- und Fehlsichtigkeitsdaten mit größtmöglicher Sicherheit ausgeschlossen ist. Dies gilt insbesondere dann, wenn die Überführung des Patienten von der Messeinrichtung bzw. den Messeinrichtungen zur Lasereinrichtung mittels einer Lagerungseinrichtung erfolgt, die mit der Messeinrichtung bzw. der Lasereinrichtung so zusammenwirkt, dass die jeweiligen Einrichtungen erkennen, ob der Patient in der jeweiligen Position zum Vermessen bzw. Einbringen der Laserstrahlung ist. Mit einem Verbringen des Patienten von der Messeinrichtung zur Lasereinrichtung kann dabei zugleich auch die Übertragung der Mess- und Fehlsichtigkeitsdaten an die Behandlungsvorrichtung erfolgen.

Es ist sichergestellt, dass die Planungseinrichtung immer den zum Patienten gehörenden Steuerdatensatz erzeugt, und eine irrtümliche Verwendung eines falschen Steuerdatensatzes für einen Patienten ist so gut wie ausgeschlossen.

Diesem Aspekt trägt auch eine weitere mögliche Ausgestaltung der Erfindung Rechnung, gemäß der der Steuerdatensatz zur Behandlungsvorrichtung übertragen wird und weiter vorzugsweise ein Betrieb der Lasereinrichtung gesperrt ist, bis an der Lasereinrichtung ein gültiger Steuerdatensatz vorliegt. Ein gültiger Steuerdatensatz kann prinzipiell ein Steuerdatensatz sein, der zur Verwendung mit der Lasereinrichtung der Behandlungsvorrichtung geeignet ist. Zusätzlich kann die Gültigkeit aber auch daran geknüpft werden, dass weitere Prüfungen vorgenommen werden, beispielsweise ob im Steuerdatensatz zusätzlich niedergelegte Angaben über den Patienten, beispielsweise eine Patientenidentifikationsnummer, mit anderen Angaben übereinstimmen, die beispielsweise an der Behandlungsvorrichtung separat eingegeben wurden, sobald der Patient in der korrekten Stellung für den Betrieb der Lasereinrichtung ist. Die Übertragung kann mittels Speicherchips (z.B. per USB oder memory stick), Magnetspeichern (z.B. Disketten), per Funk (z.B. WLAN, UMTS, Bluetooth) oder drahtgebunden (Z.B. USB, Firewire, RS232, CAN-Bus, Ethernet etc.) erfolgen.

Zur Erzeugung des isolierten Volumens wird regelmäßig der Fokus der fokussierten gepulsten Laserstrahlung dreidimensional lageverstellt. Es wird deshalb in der Regel eine zweidimensionale Ablenkung der Laserstrahlung, z.B. durch Scanspiegel, mit gleichzeitiger Fokusverstellung in der dritten Raumrichtung, z.B. durch ein Teleskop, kombiniert. Die Einstellung der Lage des Fokus ist natürlich für die Genauigkeit, mit der die das Volumen isolierende Schnittfläche erzeugt werden kann, ausschlaggebend. Es hat sich als zweckmäßig erwiesen, ein Kontaktglas zu verwenden, das auf das Auge aufgesetzt wird und dieses fixiert. Ein solches Kontaktglas ist auch bei den eingangs erwähnten Laserkeratomen, die bei der fs-LASIK-Methode verwendet werden, üblich. Das Kontaktglas hat dabei regelmäßig auch die Funktion, der Hornhautvorderfläche eine bekannte Form zu verleihen. Bei den bislang bekannten Laserkeratomen ist diese Form eine Ebene, d.h. das Auge wird zum Betrieb des Laserkeratoms im Bereich der Hornhaut flachgedrückt. Da dies für den Patienten relativ unangenehm ist, wurde für Ansätze, die ein Volumen in der Hornhaut isolieren, bereits ein gekrümmtes Kontaktglas beschrieben. Ein solches Kontaktglas verleiht der Hornhautvorderfläche dann eine bekannte Krümmung. Die Krümmung hat natürlich zwangsläufig eine Verformung der Augenhornhaut zur Folge. Diese Verformung ist umso größer, je stärker die Krümmung der dem Auge zugewandten Kontaktfläche des Kontaktglases von der tatsächlichen Hornhautkrümmung des Auges des Patienten abweicht. Um möglichst mit einer einheitlichen Kontaktglaskrümmung arbeiten zu können, ist es deshalb vorteilhaft, die Hornhautverformung, die bei der Anwendung eines gekrümmten Kontaktglases auftritt, im Steuerdatensatz zu berücksichtigen, damit unabhängig vom Grad der Verformung die gewünschte Grenzfläche für das definierte Volumen im freien, d.h. nicht durch das Kontaktglas verformte Auge vorliegt. Es ist deshalb zu bevorzugen, dass bei der Erzeugung des Steuerdatensatzes, der das Muster der Zielpunkte enthält, eine solche Verformung der Hornhaut des Auges berücksichtigt wird, die während des Einstrahlens der gepulsten Laserstrahlung vorliegt, insbesondere durch das erwähnte Kontaktglas.

Dieser Ansatz erlaubt es nicht nur eine möglichst einheitliche Kontaktglaskrümmung zu verwenden, sondern erreicht zugleich eine höhere Güte der Fehlsichtigkeitskorrektur. Bei den bekannten Laserkeratomen ist eine solche Berücksichtigung der Verformung gerade nicht erforderlich, da dort das Auge durch das Kontaktglas an der Hornhaut flachgedrückt wird. Die Erzeugung der für die LASIK-Operation erforderlichen Lamelle geschieht dort, indem einfach in einer Ebene parallel zum Kontaktglas optische Durchbrüche erzeugt werden.

Der erzeugte Steuerdatensatz kann direkt zur Ansteuerung der Behandlungsvorrichtung verwendet werden. Es ist jedoch zweckmäßig, dem behandelnden Arzt eine Eingriffsmöglichkeit zu geben, damit er zum einen den Steuerdatensatz überprüfen kann und zum anderen Spezialfälle oder Sonderwünsche berücksichtigen kann. Ein möglicher Sonderwunsch ist beispielsweise die Lage des Schnittes, über den das isolierte Volumen aus der Hornhaut entnommen werden soll. Hier vertreten oft Ophthalmologen unterschiedliche Meinungen. Aber auch aus haftungsrechtlichen Gründen kann es für den Arzt wünschenswert sein, eine Eingriffsmöglichkeit zu haben. Es ist deshalb für die Vorrichtung als mögliche Ausgestaltung zweckmäßig, dass die Planungseinrichtung eine Anzeigeeinrichtung zur visuellen Darstellung von Steuerdaten des Steuerdatensatzes und eine Eingabeeinrichtung zum nachträglichen Verändern oder Beeinflussen des Steuerdatensatzes, aufweist.

Optische Systeme sind in der Regel nicht perfekt. Dies gilt natürlich auch für die Fokussierung der Laserstrahlung in die Hornhaut. Hier kann beispielsweise eine Bildfeldkrümmung auftreten, die zur Folge hat, dass vermeintlich in einer Ebene positionierte Fokuslagen tatsächlich gar nicht in einer Ebene liegen, sondern in einer gekrümmten Fläche. Bei den bekannten Laserkeratomen spielt dieser Gesichtspunkt keine Rolle, da die Erzeugung des die Lamelle freilegenden Schnittes für die optische Qualität der Korrektur ohne Auswirkung ist. Die eigentliche Korrektur wird ausschließlich durch das mit dem Ablationslaser verdampfte Volumen der freigelegten Hornhaut bestimmt. Eine Fehlerkorrektur beispielsweise hinsichtlich einer Bildfeldkrümmung ist deshalb im Stand der Technik, insbesondere betreffend Laserkeratome, nicht von Interesse.

Da nun das zu entnehmende Volumen gänzlich durch die Fokussierung der gepulsten Laserstrahlung definiert wird, ist es zweckmäßig, dass die Planungseinrichtung zur Erzeugung des Steuerdatensatzes optische Fokuslagenfehler, die beim Fokussieren der gepulsten Laserstrahlung zu einer Abweichung zwischen vorgegebener und tatsächlicher Lage der Zielpunkte führen, durch einen von der Lage des jeweiligen Zielpunktes abhängigen Vorhalts berücksichtigt und damit ausgleicht. Dieser Vorhalt kann beispielsweise dadurch ermittelt werden, dass die Planungseinrichtung auf eine Korrekturtabelle oder -funktion zugreift, welche den Fokuslagenfehler abhängig von der Lage des jeweiligen Zielpunktes angibt. Die Korrekturtabelle oder -funktion kann für den jeweiligen Gerätetyp einheitlich vorgeschrieben, oder, was aus Gründen der Präzision bevorzugt ist, für das jeweilige Gerät individuell ermittelt werden.

Analoges gilt für das Verfahren, bei dem nun für die Festlegung der Grenzfläche oder des dreidimensionalen Musters der Zielpunkte optische Fokuslagenfehler, die beim Fokussieren der gepulsten Laserstrahlung zu einer Abweichung zwischen vorgegebener und tatsächlicher Lage der Zielpunkte führen, durch einen von der Lage des jeweiligen Zielpunktes abhängigen Vorhalt berücksichtigt und damit ausgeglichen werden.

Die Grenzfläche isoliert das Volumen, wenn sie nach Einsatz der gepulsten Laserstrahlung als Schnittfläche ausgebildet ist. Die Grenzfläche hat damit automatisch anteriore und posteriore Abschnitte, wobei die Begriffe "anterior" und "posterior" hier der üblichen medizinischen Nomenklatur entsprechen. Prinzipiell ist es möglich, die Grenzfläche als Freifläche zu gestalten. Die Erzeugung des Steuerdatensatzes ist jedoch vereinfacht, wenn die Grenzfläche aus einer anterioren Teilfläche und einer posterioren Teilfläche zusammengesetzt ist. Eine der Teilflächen kann dann in konstantem Abstand zur Hornhautoberfläche ausgebildet werden. Die andere hat dann zwangsläufig keinen konstanten Abstand zur Hornhautvorderfläche. Die in konstantem Abstand zur Hornhautvorderfläche liegende Teilfläche, meist die anteriore Teilfläche, ist damit in der Regel sphärisch ausgebildet. Dies gilt auf jeden Fall dann, wenn die Augenhornhaut auf ein sphärisches Kontaktglas angedrückt wird. Die optische Korrektur erfolgt dann durch die Form der anderen Teilfläche, in der Regel der posterioren Teilfläche. Der Rechenaufwand ist dadurch erheblich vereinfacht.

Eine Möglichkeit, die Fehlsichtigkeitsdaten anzugeben, besteht darin, die Brechkraft $B_{BR}$ einer für die Fehlsichtigkeitskorrektur tauglichen Brille, die in einem Abstand $d_{HS}$ vor dem Hornhautscheitel liegen muss, um die gewünschte Fehlsichtigkeitskorrektur zu erreichen, zu bestimmen. Eine Bestimmung dieser Parameter ist gängiger Standard in der Augenoptik und ermöglicht die Verwendung weitverbreiteter und seit langem eingeführter Messeinrichtungen. Zur Erzeugen des Steuerdatensatzes wird dann lediglich auf die Fehlsichtigkeitsdaten für eine übliche Brillenkorrektur zurückgegriffen. Selbstverständlich können solche Daten auch Astigmatismuskorrekturen beinhalten. Eine übliche Formel für die Brechkraft $B_{BR}$ einer Brille ist beispielsweise die in nachfolgender Figurenbeschreibung angegebene Gleichung (1). Sie gibt den sphärischen Brechungsfehler Sph sowie den zylindrischen Brechungsfehler Cyl an und setzt natürlich für letzteren die Kenntnis der Zylinderachse $\theta$ voraus.

Zur Fehlsichtigkeitskorrektur wird mit der Behandlungsvorrichtung bzw. unter Verwendung der im beschriebenen Verfahren erzeugten Steuerdatensätze ein Volumen aus der Hornhaut entfernt. Ziel ist letztlich, die Krümmung der Hornhaut so zu ändern, dass eine Fehlsichtigkeitskorrektur erreicht ist. Eine besonders direkte exakte und einfache Berechnung der für die Korrektur zu erreichenden Krümmung der Hornhautvorderfläche ergibt sich mit folgender Gleichung:

$$R_{CV}{}^* = 1 / ( (1/R_{CV}) + B_{BR} / ( (n_c\text{-}1) (1 - d_{HS} \cdot B_{BR}))) + F.$$

In dieser Gleichung bezeichnet Rcv* dem Krümmungsradius der Hornhautvorderfläche nach Entfernung des Volumens, Rcv den Krümmungsradius der Hornhaut vor der Entfernung des Volumens (er ist in den Messdaten enthalten), $n_c$ die Brechkraft des Materials der Hornhaut (meist etwa 1,376), $d_{HS}$ den Abstand, in dem eine Brille mit erwähnte Brechkraft vor dem Hornhautscheitel liegen muss und F einen Faktor, der einen Maß für die optische Wirkung der Dickenabnahme der Augenhornhaut auf des Sehachse aufgrund der Entfernung des Volumens ist. Zu einer vereinfachten Rechnung kann der Faktor F gleich Null gesetzt werden. Möchte man eine genauere Berechnung, so kann F wie folgt berechnet werden:

$$F = (1 - 1/n_c) \cdot (d_C{}^* - d_C),$$

wobei dc bzw. dc* die Dicke der Hornhaut vor bzw. nach Entfernung des Volumens bezeichnet. Der Radius Rcv* wird dann iterativ berechnet, indem bei jedem Iterationsschritt aus der Differenz (Rcv* - Rcv) auf die Größe (dc* - dc) geschlossen wird und das entsprechende daraus erhaltene Ergebnis für die Dickenänderung bei der Berechnung von Rcv* im nächsten Iterationsschritt angewendet wird. Die iterative Berechnung kann beispielsweise abgebrochen werden, wenn zwischen zwei Iterationsschritten für F nur noch ein Unterschied besteht, der kleiner als ein Grenzwert ist.

Die erfindungsgemäße Behandlungsvorrichtung mit der Planungseinrichtung arbeitet besonders einfach, wenn, wie erwähnt, die optische Korrektur, die durch die Entfernung des Volumens bewirkt werden soll, vornehmlich durch die Krümmung einer nicht in konstantem Abstand zur Hornhautvorderfläche liegenden Teilfläche, welche das Volumen begrenzt, gebildet ist. Zweckmäßigerweise wird man dazu die posteriore Teilfläche wählen, da diese Teilfläche dann zumindest bei Myopiekorrektur den oben erwähnten Krümmungsradius vermindert um den konstanten Abstand zwischen anteriorer Teilfläche und Hornhautvorderfläche hat.

Der Steuerdatensatz stellt eine Datei bereit, die einen vollautomatischen Ablauf des operativen Eingriffes hinsichtlich der Steuerung der Behandlungsvorrichtung bzw. ein entsprechenden Betrieb der Behandlungsvorrichtung ermöglicht. Der Steuerdatensatz gibt dazu der Lasereinrichtung die Zielpunkte vor, auf die der fokussierte Laserstrahl zur Abgabe von Laserpulsen gerichtet werden muss. Der Fokus der fokussierten Laserstrahlung wird dann so verstellt, dass er in einer Bahnkurve über die vorgegebenen Zielpunkte läuft. Berechnungstechnisch bzw. hinsichtlich der Verstellgeschwindigkeit ist es dabei besonders günstig, wenn die Bahnkurve von einer Spirale vorliegt. Im Fall der erwähnten anterioren bzw. posterioren Teilfläche ist dann für jede Teilfläche eine Spirale vorgegeben. Der Verlauf des Fokus entlang einer Spirale ermöglicht einen Betrieb der entsprechenden Ablenkeinrichtung der Behandlungsvorrichtung nahe der Grenzfrequenz, da z.B. beim Schreiben einer Spirale zwei Galvanometerscanner jeweils nahe oder an ihrer Grenzfrequenz betrieben werden können.

Grundsätzlich muss bei der Definition der Bahn dafür gesorgt werden, dass die Laserpulse auf dieser Bahn abgegeben werden. Die Zielpunkte definieren dann Stützstellen in der Bahn. Die Dichte, mit der die Zielpunkte die Bahn vorgeben, kann, muss aber nicht zwangsläufig der Dichte entsprechen, mit der die Punkte auf der Bahn angeordnet sind, auf die jeweils ein Puls der Laserstrahlung abgegeben wird. Es ist im Gegenteil sogar zu bevorzugen, dass die Zielpunkte nur eine Teilmenge derjenigen Punkte darstellen, auf die Laserpulse abgegeben werden. Zum einen ist der Steuerdatensatz dann in seiner Datenmenge drastisch verringert, zum anderen vereinfacht sich der Rechenaufwand in all den Schritten, in denen nicht mit einer funktionellen Beschreibung der Bahnkurve in der Grenzfläche gearbeitet werden soll oder kann, sondern in denen die Zielpunkte einzeln prozessiert werden müssen. Ein Beispiel hierfür ist die erwähnte Korrektur hinsichtlich Bildfeldkrümmung.

Es ist für die Vorrichtung deshalb bevorzugt, dass die Lasereinrichtung die fokussierte Laserstrahlung entlang einer Bahn über das Muster der Zielpunkte verstellt, wobei Pulse der gepulsten Laserstrahlung in die Hornhaut auf Punkte abgegeben sind, die auf der Bahn zwischen den Zielpunkten liegen.

Analoges gilt für das beschriebene Verfahren, nämlich dass der Steuerdatensatz für eine Lasereinrichtung vorgesehen wird, welche die fokussierte Laserstrahlung entlang einer Bahn über das Muster der Zielpunkte verstellt, wobei der Steuerdatensatz so erzeugt ist, dass die Zielpunkte im Muster eine Teilmenge der Punkte darstellen, auf die die Lasereinrichtung die gepulste Laserstrahlung abgibt. Der Steuerdatensatz ist somit auf die mögliche Verstellgeschwindigkeit der Lasereinrichtung abgestimmt.

Im Ergebnis wird in der Lasereinrichtung für die Verstellung der Fokuslage eine andere Frequenz der Stützstellen vorgegeben bzw. angewendet, als sie bei der Erzeugung der Laserpulse auftritt. Natürlich enthält der Steuerdatensatz per se keine Angabe über die Frequenz, auch wenn dies möglich ist. Aufgrund der maximalen Verstellgeschwindigkeit bzw. der höchsten Signalfrequenzen, die bei der Verstellung des Fokus der Laserstrahlung Anwendung findet, entspricht die Vorgabe der Zielpunkte natürlich einer Bahngeschwindigkeit bzw. einer Verstellgeschwindigkeit in den jeweiligen Koordinaten, die zur Beschreibung verwendet werden. Der räumliche Abstand der Zielpunkt in Kombination mit der Bahngeschwindigkeit und der Laserpulsfrequenz, die von der Lasereinrichtung realisiert werden kann, führt nun in der bevorzugten Ausgestaltung dazu, dass automatisch auch Laserpulse zu Zeitpunkten abgegeben werden, zu denen die Verstellung des Fokus von einem Zielpunkt zum nächsten erfolgt. Dieser Ansatz hat den Vorteil, dass im Betrieb der Behandlungsvorrichtung die Zielpunkte mit einer Frequenz vorgegeben sind/werden, die kleiner als sie Frequenz ist, mit der die Pulse der gepulsten Laserstrahlung von der Lasereinrichtung in die Hornhaut abgegeben werden/sind.

Selbstverständlich bedeutet die Vorgabe von Zielpunkten im Steuerdatensatz nicht, dass an diesen Zielpunkten eine Verstellgeschwindigkeit gleich Null vorliegen muss, wenn der Laserpuls auf dem Zielpunkt abgegeben wird. Im Sinne einer schnellen Erzeugung der Schnittfläche an der festgelegten Grenzfläche des definierten Volumens ist es vorteilhaft, wenn die Synchronisation von Verstellung der Fokuslage und Abgabe der Laserpulse derart erreicht ist, dass ein Laserpuls bei kontinuierlicher Ablenkung des Fokus abgegeben wird und dennoch am Zielpunkt in die Hornhaut trifft. Die gepulste Laserstrahlung wird also bei fortlaufender Verstellung der Fokuslage, z.B. bei bewegten Scanspiegeln, appliziert. Diese Ausgestaltung bedingt eine systematischen Unterschied gegenüber bekannten shot files für Ablationslaser, bei denen ein Schuss des Ablationslasers immer erst dann abgegeben wird, wenn die Ablenkung des Laserstrahls ruhend auf einen bestimmten Punkt zielt. Der Umfang des beanspruchten Gegenstands der Erfindung wird durch die Ansprüche definiert. Beschriebene Verfahren und Verfahrensschritte sind ausdrücklich nicht Teil der beanspruchten Erfindung.

Die Erfindung wird nachfolgend unter Bezugnahme auf die Zeichnung beispielhalber noch näher erläutert. In den Zeichnungen zeigt:

Fig. 1      eine Schemadarstellung einer Behandlungsvorrichtung bzw. eines Behandlungsgerätes zur Fehlsichtigkeitskorrektur,

Fig. 1a      eine Schemadarstellung hinsichtlich des Aufbaus des Behandlungsgerätes der Fig. 1,

Fig. 2      eine Prinzipdarstellung zur Einbringung gepulster Laserstrahlung in das Auge bei der Fehlsichtigkeitskorrektur mit dem Behandlungsgerät der Fig. 1,

Fig. 3      eine weitere Schemadarstellung des Behandlungsgerätes der Fig. 1,

Fig. 4      in Teilfiguren (a), (b) und (c) schematische Schnittdarstellungen zur Verdeutlichung des Korrekturbedarfes am menschlichen Auge bei Fehlsichtigkeit,

Fig. 5      eine schematische Schnittdarstellung durch die Augenhornhaut mit Darstellung eines zur Fehlsichtigkeitskorrektur zu entfernenden Volumens,

Fig. 6      ein Schnitt durch die Augenhornhaut nach Entfernung des Volumens der Fig. 5,

Fig. 7      eine Schnittdarstellung ähnlich der Fig. 5,

Fig. 8      eine schematische Schnittdarstellung durch die Augenhornhaut zur Veranschaulichung der Volumenentnahme,

Fig. 9      eine Draufsicht auf eine spiralförmige Bahnkurve, die zur Isolierung des Volumens der Fig. 5, 7 und 8 verwendet wird,

Fig. 10      eine vergrößerte Darstellung der Bahnkurve der Fig. 9,

Fig. 11      eine alternative Bahnkurve für die Korrektur auch zylindrischer Fehlsichtigkeiten,

Fig. 12      eine schematische Darstellung zur Erläuterung der Funktion eines Kontaktglases im Behandlungsgerät der Fig. 1,

Fig. 13 bis 15      schematische Darstellungen hinsichtlich der Wirkungen des Kontaktglases durch Verformung der Augenhornhaut,

Fig. 16 und 17      Schemadarstellung hinsichtlich der Approximation der das Volumen posterior begrenzenden Fläche im Falle auch zylindrischer Fehldichtigkeitskorrekturen,

Fig. 18      eine schematische Darstellung zu einer bei der Ermittlung von Steuerdaten für das Behandlungsgerät der Fig. 1 verwendeten Bildfeldkrümmungskorrektur und

Fig. 19      eine schematische Darstellung des Ablaufes bei der Vorbereitung und Durchführung einer Fehlsichtigkeitskorrektur.

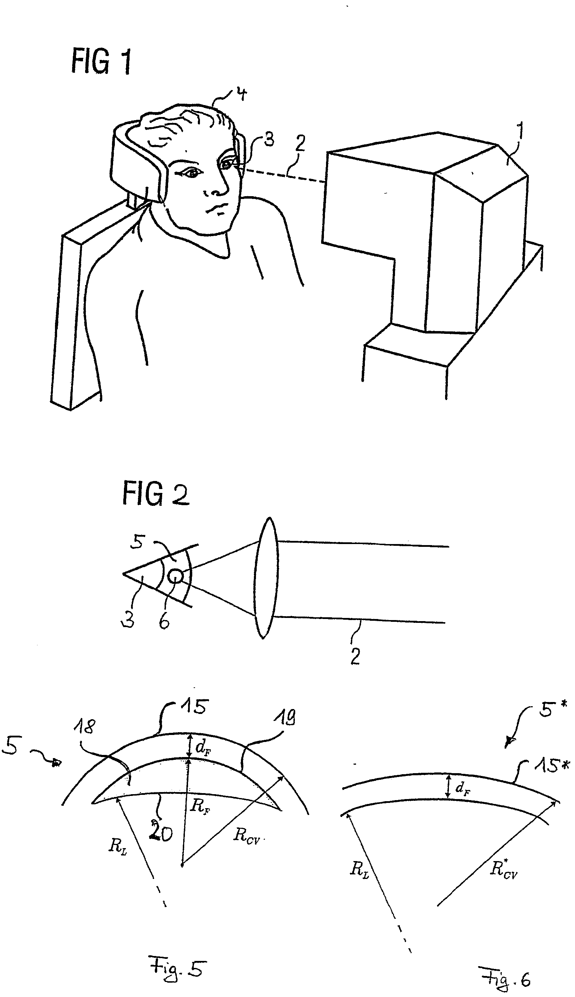

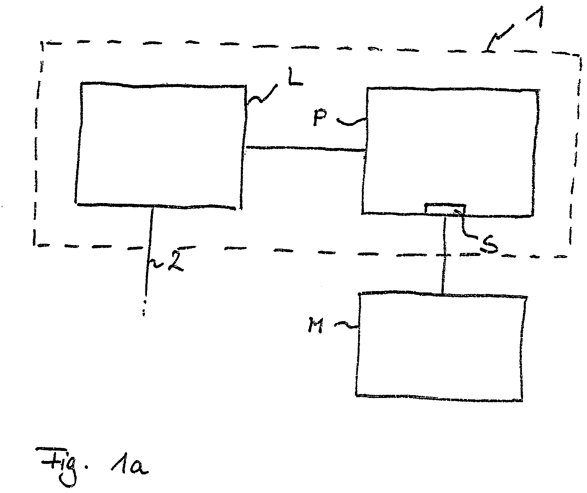

Figur 1 zeigt ein Behandlungsgerät 1 für ein augenchirurgisches Verfahren, das dem in der EP 1159986 A1 bzw. der US 5549632 beschriebenen ähnelt. Das Behandlungsgerät 1 bewirkt mittels ein Behandlungs-Laserstrahlung 2 eine Fehlsichtigkeitskorrektur an einem Auge 3 eines Patienten 4. Die Fehlsichtigkeit kann Hyperopie, Myopie, Presbyopie, Astigmatismus, gemischten Astigmatismus (Astigmatismus, bei dem in einer Richtung Hyperopie und in einer rechtwinklig dazu liegenden Richtung Myopie vorliegt), asphärische Fehler und Aberrationen höherer Ordnung umfassen. Die Behandlungs-Laserstrahlung 2 wird in der beschriebenen Ausführungsform als gepulster in das Auge 3 fokussierter Laserstrahl aufgebracht. Die Pulsdauer liegt dabei z.B. im Femtosekundenbereich, und die Laserstrahlung 2 wirkt mittels nicht-linearer optischer Effekte in der Hornhaut. Der Laserstrahl weist z.B. 50 bis 800 fs kurze Laserpulse (bevorzugt 100 - 400 fs) mit einer Pulswiederholfrequenz zwischen 10 und 500 kHz auf. Die Baugruppen des Gerätes 1 werden im beschriebenen Ausführungsbeispiel von einer integrierten Steuereinheit gesteuert, die aber natürlich auch eigenständig ausgebildet sein kann.

Vor dem Einsatz des Behandlungsgerätes wird die Fehlsichtigkeit des Auges 3 mit einer oder mehreren Messeinrichtungen vermessen.

Figur 1a zeigt schematisch das Behandlungsgerät 1. Es weist in dieser Variante mindestens zwei Einrichtungen oder Module auf. Eine Lasereinrichtung L gibt den Laserstrahl 2 auf das Auge 3 ab. Der Betrieb der Lasereinrichtung L erfolgt dabei vollautomatisch, d.h. die Lasereinrichtung L startet auf ein entsprechendes Startsignal hin die Ablenkung des Laserstrahls 2 und erzeugt dabei Schnittflächen, die auf noch zu beschreibende Art und Weise aufgebaut sind und ein Volumen in der Augenhornhaut isolieren. Die den Betrieb erforderlichen Steuerdaten empfängt die Lasereinrichtung L zuvor von einer Planungseinrichtung P als Steuerdatensatz über nicht näher bezeichnete Steuerleitungen. Die Übertragung findet vor dem Betrieb der Lasereinrichtung L statt. Natürlich kann Kommunikation auch drahtlos erfolgen. Alternativ zu einer direkten Kommunikation ist es auch möglich, die Planungseinheit P räumlich getrennt von der Lasereinheit L anzuordnen und einen entsprechenden Datenübertragungskanal vorzusehen.

Vorzugsweise wird der Steuerdatensatz zum Behandlungsgerät 1 übertragen und weiter vorzugsweise ist ein Betrieb der Lasereinrichtung L gesperrt, bis an der Lasereinrichtung L ein gültiger Steuerdatensatz vorliegt. Ein gültiger Steuerdatensatz kann ein Steuerdatensatz sein, der prinzipiell zur Verwendung mit der Lasereinrichtung L der Behandlungsvorrichtung 1 geeignet ist. Zusätzlich kann die Gültigkeit aber auch daran geknüpft werden, dass weitere Prüfungen bestanden werden, beispielsweise ob im Steuerdatensatz zusätzlich niedergelegte Angaben über das Behandlungsgerät 1, z. B. eine Geräteseriennummer, oder den Patienten, z. B. eine Patientenidentifikationsnummer, mit anderen Angaben übereinstimmen, die beispielsweise an der Behandlungsvorrichtung ausgelesen oder separat eingegeben wurden, sobald der Patient in der korrekten Stellung für den Betrieb der Lasereinrichtung L ist.

Die Planungseinheit P erzeugt den Steuerdatensatz, der der Lasereinheit L zur Ausführung der Operation zur Verfügung gestellt wird, aus Messdaten und Fehlsichtigkeitsdaten, die für das zu behandelnde Auge ermittelt wurden. Sie werden der Planungseinheit P über eine Schnittstelle S zugeführt und stammen im dargestellten Ausführungsbeispiel aus einer Messeinrichtung M, die das Auge des Patienten 4 zuvor vermessen hat. Natürlich kann die Messeinrichtung M auf beliebige Art und Weise die entsprechenden Mess- und Fehlsichtigkeitsdaten an die Planungseinrichtung P übermitteln.

Die Übertragung kann mittels Speicherchips (z.B. per USB oder memory stick), Magnetspeichern (z.B. Disketten), per Funk (z.B. WLAN, UMTS, Bluetooth) oder drahtgebunden (Z.B. USB, Firewire, RS232, CAN-Bus, Ethernet etc.) erfolgen. Gleiches gilt natürlich hinsichtlich der Datenübertragung zwischen Planungseinrichtung P und Lasereinrichtung L.

Eine direkte Funk- oder Draht-Verbindung der Messeinrichtung M mit der Behandlungseinrichtung 1 hinsichtlich der Datenübertragung, die in einer Variante verwendet werden kann, hat den Vorteil, dass die Verwendung falscher Mess- und Fehlsichtigkeitsdaten mit größtmöglicher Sicherheit ausgeschlossen ist. Dies gilt insbesondere dann, wenn die Überführung des Patienten von der Messeinrichtung M bzw. den Messeinrichtungen zur Lasereinrichtung L mittels einer (in der Figur nicht dargestellten) Lagerungseinrichtung erfolgt, die mit der Messeinrichtung M bzw. der Lasereinrichtung L so zusammenwirkt, dass die jeweiligen Einrichtungen erkennen, ob der Patient 4 in der jeweiligen Position zum Vermessen bzw. Einbringen der Laserstrahlung 2 ist. Mit einem Verbringen des Patienten 4 von der Messeinrichtung M zur Lasereinrichtung L kann dabei zugleich auch die Übertragung der Mess- und Fehlsichtigkeitsdaten an die Behandlungsvorrichtung 1 erfolgen.

Es ist vorzugsweise durch geeignete Mittel sichergestellt, dass die Planungseinrichtung P immer den zum Patienten 4 gehörenden Steuerdatensatz erzeugt, und eine irrtümliche Verwendung eines falschen Steuerdatensatzes für einen Patienten 4 ist so gut wie ausgeschlossen.

Die Wirkungsweise des Laserstrahls 2 ist in Figur 2 schematisch angedeutet. Der Behandlungs-Laserstrahl 2 wird mittels einer nicht näher bezeichneten Optik in die Hornhaut 5 des Auges 6 fokussiert. Dadurch entsteht in der Hornhaut 5 ein Fokus, der einen Spot 6 überdeckt und in dem die Laserstrahlungsenergiedichte so hoch ist, dass in Kombination mit der Pulslänge ein nicht-linearer Effekt im Auge auftritt. Beispielsweise kann jeder Puls der gepulsten Laserstrahlung 2 am jeweiligen Spot 6 einen optischen Durchbruch in der Augenhornhaut 5 erzeugen, welcher wiederum eine in Figur 2 schematisch angedeutete Plasmablase initiiert. Dadurch wird dieses Laserpulses in der Hornhaut 5 Gewebe getrennt. Bei Entstehung einer Plasmablase umfasst die Gewebeschichttrennung ein größeres Gebiet, als den Spot 6, welchen der Fokus der Laserstrahlung 2 überdeckt, obwohl die Bedingungen zur Erzeugung des Durchbruches nur im Fokus erreicht werden. Damit von jedem Laserpuls ein optischer Durchbruch erzeugt wird, muss die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt.

Alternativ kann ein gewebetrennender Effekt durch die gepulste Laserstrahlung auch dadurch erzeugt werden, dass mehrere Laserstrahlungspulse im einen Bereich abgegeben werden, wobei sich für mehrere Laserstrahlungspulse die Spots 6 überlappen. Es wirken dann mehrere Laserstrahlungspulse zusammen, um einen gewebetrennenden Effekt zu erreichen.

Die Art der Gewebetrennung, die das Behandlungsgerät 1 einsetzt, ist jedoch für die nachfolgende Beschreibung nicht weiter relevant, wesentlich ist lediglich, dass dazu gepulste Behandlungs-Laserstrahlung 2 verwendet wird. Beispielsweise kann ein Behandlungsgerät 1 verwendet werden, wie sie in der WO 2004/032810 A2 beschrieben ist. Wesentlich ist weiter, dass eine Vielzahl von Laserpulsfoki im Gewebe eine Schnittfläche ausbildet, deren Form vom Muster abhängt, mit dem die Laserpulsfoki im Gewebe angeordnet sind/werden. Das Muster gibt Zielpunkte für die Fokuslage vor, an denen ein oder mehrere Laserpuls(e) abgegeben wird(werden), und definiert die Form und Lage der Schnittfläche. Für die nachfolgend erläuterten Verfahren und Vorrichtungen ist das Muster der Zielpunkte von Bedeutung und wird noch näher beschrieben werden.

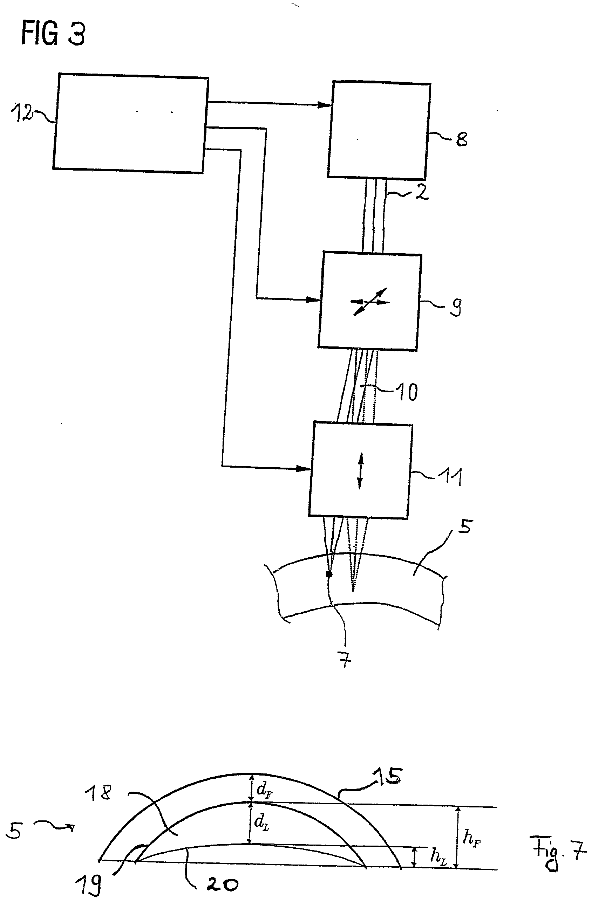

Um nun eine Fehlsichtigkeitskorrektur auszuführen, wird mittels der gepulsten Laserstrahlung aus einem Gebiet innerhalb der Hornhaut 5 Material entfernt, indem dort Gewebeschichten getrennt werden, die das Material isolieren und dann eine Materialentnahme ermöglichen. Die Materialentfernung bewirkt eine Volumenänderung in der Hornhaut, welche eine Änderung der optischen Abbildungswirkung der Hornhaut 5 zur Folge hat, die genau so bemessen ist, dass damit die zuvor ermittelte Fehlsichtigkeit möglichst korrigiert ist/wird. Zur Isolierung des zu entfernenden Volumens wird der Fokus der Laserstrahlung 2 auf Zielpunkte in der Hornhaut 5 gerichtet, in der Regel in einem Bereich, der unterhalb des Epithels und der Bowman'schen Membran sowie oberhalb der Decemetschen Membran und des Endothels liegt. Das Behandlungsgerät 1 weist dazu einen Mechanismus zum Verstellen der Lage des Fokus der Laserstrahlung 2 in der Hornhaut 5 auf. Dies ist schematisch in Figur 3 gezeigt.

In Figur 3 sind Elemente des Behandlungsgeräts 1 nur insoweit eingetragen, als sie zum Verständnis der Fokusverstellung erforderlich sind. Die Laserstrahlung 2 wird, wie bereits erwähnt, in einem Fokus 7 in der Hornhaut 5 gebündelt, und die Lage des Fokus 7 in der Hornhaut wird verstellt, so dass zur Schnittflächenerzeugung an verschiedenen Stellen fokussiert Energie aus Laserstrahlungspulsen in das Gewebe der Hornhaut 3 eintragen wird. Die Laserstrahlung 2 wird von einem Laser 8 als gepulste Strahlung bereitgestellt. Ein xy-Scanner 9, der in einer Variante durch zwei im Wesentlichen orthogonal ablenkende Galvanometerspiegel realisiert ist, lenkt den vom Laser 8 kommenden Laserstrahl zweidimensional ab, so dass nach dem xy-Scanner 9 ein abgelenkter Laserstrahl 10 vorliegt. Der xy-Scanner 9 bewirkt somit eine Verstellung der Lage des Fokus 7 im Wesentlichen senkrecht zur Haupteinfallsrichtung der Laserstrahlung 2 in die Hornhaut 5. Zur Verstellung der Tiefenlage ist neben dem xy-Scanner 9 ein z-Scanner 11 vorgesehen, der beispielsweise als verstellbares Teleskop ausgebildet ist. Der z-Scanner 11 sorgt dafür, dass die z-Position der Lage des Fokus 7, d.h. dessen Position auf der optischen Achse des Einfalls verändert wird. Der z-Scanner 11 kann dem xy-Scanner 9 nach- oder vorgeordnet sein. Die nachfolgend mit x, y, z bezeichneten Koordinaten beziehen sich also auf die Ablenkung der Lage des Fokus 7.

Für das Funktionsprinzip des Behandlungsgerätes 1 ist die Zuordnung der einzelnen Koordinaten zu den Raumrichtungen nicht wesentlich, der einfacheren Beschreibung halber ist jedoch nachfolgend mit z immer die Koordinate entlang der optischen Achse des Einfalls der Laserstrahlung 2 bezeichnet, und x sowie y bezeichnen zwei zueinander orthogonale Koordinaten in einer Ebene senkrecht zur Einfallsrichtung des Laserstrahls. Dem Fachmann ist natürlich bekannt, dass eine dreidimensionale Beschreibung der Lage des Fokus 7 in der Hornhaut 5 auch durch andere Koordinatensysteme erfolgen kann, insbesondere muss es sich nicht um ein rechtwinkliges Koordinatensystem handeln. Dass der xy-Scanner 9 um zueinander rechtwinklige Achsen ablenkt ist also nicht zwingend, vielmehr kann jeder Scanner verwendet werden, der in der Lage ist, den Fokus 7 in einer Ebene zu verstellen, in der die Einfallsachse der optischen Strahlung nicht liegt. Somit sind auch schiefwinklige Koordinatensysteme möglich.

Weiter können auch nicht-kartesische Koordinatensysteme zur Beschreibung bzw. Steuerung der Lage des Fokus 7 verwendet werden, wie dies nachfolgend auch noch erläutert wird. Beispiele für solche Koordinatensysteme sind Kugelkoordinaten (auch als sphärische Koordinaten bezeichnet) sowie zylindrische Koordinaten.

Zur Steuerung der Lage des Fokus 7 werden der xy-Scanner 9 sowie der z-Scanner 11, die gemeinsam ein konkretes Beispiel einer dreidimensionalen Fokusverstelleinrichtung realisieren, von einem Steuergerät 12 über nicht näher bezeichnete Leitungen angesteuert. Gleiches gilt für den Laser 8. Das Steuergerät 3 sorgt für einen geeignet synchronen Betrieb des Lasers 8 sowie der dreidimensionalen Fokusverstelleinrichtung, exemplarisch realisiert durch den xy-Scanner 9 sowie den z-Scanner 11, so dass die Lage des Fokus 7 in der Hornhaut 5 so verstellt wird, dass letztendlich ein Material bestimmten Volumens isoliert wird, wobei die spätere Volumenentfernung eine gewünschte Fehlsichtigkeitskorrektur bewirkt.

Das Steuergerät 12 arbeitet nach vorgegebenen Steuerdaten, welche die Zielpunkte für die Fokusverstellung vorgeben. Die Steuerdaten sind in der Regel in einem Steuerdatensatz zusammengefasst. Dieser gibt in einer Ausführungsform die Koordinaten der Zielpunkte als Muster vor, wobei die Reihenfolge der Zielpunkte im Steuerdatensatz die Aneinanderreihung der Fokuslagen und damit letztlich eine Bahnkurve (hier auch verkürzt als Bahn bezeichnet) festlegt. Der Steuerdatensatz enthält in einer Ausführungsform die Zielpunkte als konkrete Stellwerte für den Fokuslagenverstellmechanismus, z.B. für den xy-Scanner 9 und den z-Scanner 11. Zur Vorbereitung des augenchirurgischen Verfahrens, also bevor das eigentliche Operationsverfahren ausgeführt werden kann, werden die Zielpunkte und vorzugsweise auch deren Reihenfolge im Muster bestimmt. Es muss eine Vorplanung des operativen Eingriffes dahingehend erfolgen, dass die Steuerdaten für das Behandlungsgerät 1 ermittelt werden, deren Anwendung dann eine für den Patienten 4 optimale Fehlsichtigkeitskorrektur erreicht.

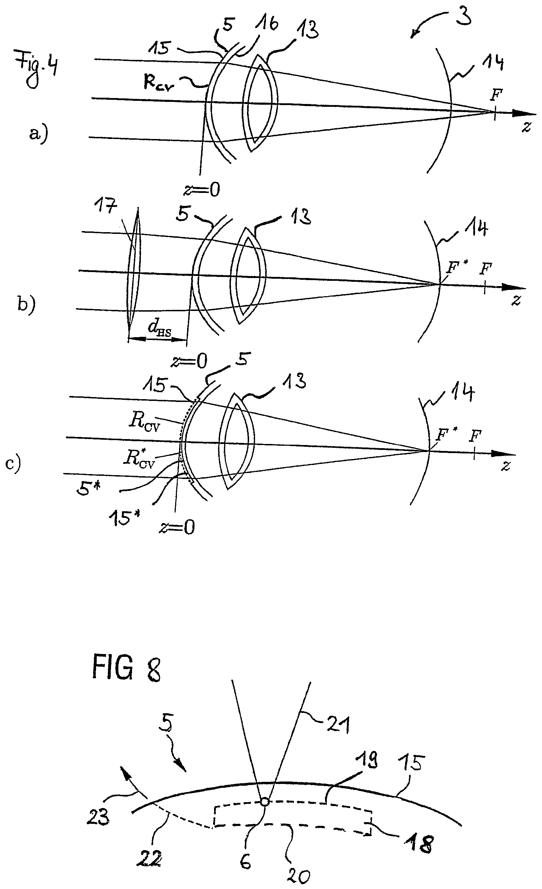

Zuerst gilt es das aus in der der Hornhaut 5 zu isolierende und später zu entfernende Volumen festzulegen. Wie bereits anhand Fig. 1a geschildert bedarf es dazu einer Feststellung des Korrekturbedarfs. Figur 4 zeigt in Teilfiguren a), b) und c) die optischen Verhältnisse am Auge 3 des Patienten 4. Ohne Fehlsichtigkeitskorrektur liegt die in Teilfigur a) gezeigte Situation vor. Die Hornhaut 5 bewirkt zusammen mit der Augenlinse 13 eine Fokussierung eines im Unendlichen liegenden Gegenstandes in einen Fokus F, der auf der z-Achse hinter der Netzhaut 14 liegt. Die abbildende Wirkung rührt dabei zum einen von der bei nicht-akkommodiertem Auge entspannten Augenlinse 13 sowie zum anderen von der Augenhornhaut 5 her, die im Wesentlichen durch eine Hornhautvorderfläche 15 sowie eine Hornhautrückseite 16 definiert ist und aufgrund ihrer Krümmung ebenfalls eine abbildende Wirkung hat. Die optische Wirkung der Hornhaut 5 ist durch den Krümmungsradius Rcv der Hornhautvorderfläche bedingt. Teilfigur a) stellt die Fehlsichtigkeit nur exemplarisch dar, real können die oben erwähnten komplexeren Fehlsichtigkeiten vorliegen. Für sie gilt die nachfolgende Beschreibung jedoch ebenfalls, allerdings können die angegebenen Gleichungen dann mitunter eine zusätzliche Winkelabhängigkeit beinhalten, auch wenn darauf nicht ausdrücklich hingewiesen wird.

Zur Fehlsichtigkeitskorrektur wird bekannter Weise, wie in Teilfigur b) der Figur 4 dargestellt, eine Vorsatz-Linse 17 in Form einer Brille im Abstand $d_{HS}$ vom Scheitelpunkt der Hornhaut 5 vor das Auge 3 gesetzt. Die Linse 17 der Brille ist in ihrer Brechkraft $B_{BR}$ so angepasst, dass sie den Fernpunkt des gesamten Systems, d.h. aus Brille und Auge, vom Fokuspunkt F zum korrigierten Fokuspunkt F* verschiebt, der auf der Netzhaut 14 liegt.

Hinsichtlich der in dieser Beschreibung verwendeten Nomenklatur sei angemerkt, dass durch die Anfügung eines Sterns an Größen verdeutlicht wird, dass es sich um Größen handelt, die nach einer Korrektur erhalten werden. Der Fokus F* ist also derjenige Fokus, der nach der optischen Korrektur vorliegt, die in der Teilfigur b) der Figur 4 durch die Linse 17 der Brille erreicht wird.

Unter der gerechtfertigten Annahme, dass eine Dickenänderung der Hornhaut 5 im Wesentlichen den Krümmungsradius der Luft zugewandten Hornhaut-Vorderseite 15 modifiziert, nicht aber den Krümmungsradius der dem Augeninneren zugewandten Hornhautrückseite 16, wird durch die Volumenentfernung der Krümmungsradius Rcv der Hornhautvorderseite 15 modifiziert. Die um das Volumen verminderte Hornhaut 5 hat eine derart geänderte Abbildungswirkung, dass der dann korrigierte Fokus F* auf der Netzhaut 14 liegt. Nach der Korrektur liegt eine veränderte Hornhautvorderfläche 15* vor, und es ist eine Fehlsichtigkeitskorrektur auch ohne Brille erreicht.

Zur Bestimmung des Musters der Zielpunkte wird deshalb die zu erreichende Krümmung der modifizierten Hornhautvorderfläche 15* ermittelt. Dabei ist Ausgangspunkt die Brechkraft der Linse 17 der Brille, da die Ermittlung der entsprechenden Parameter ein Standardverfahren in der Augenoptik ist. Für die Brechkraft $B_{BR}(\varphi)$ der Linse 17 der Brille gilt folgende Formel:

$$(1) \qquad B_{BR}(\varphi) = Sph + Cyl \cdot \sin^2(\varphi - \theta).$$

In dieser Gleichung bezeichnen Sph und Cyl die zu realisierenden Korrekturwerte sphärischen bzw. astigmatischen Brechungsfehler und $\theta$ die Lage der Zylinderachse der zylindrischen (astigmatischen) Fehlsichtigkeit, wie sie dem Fachmann in der Optometrie bekannt sind. Der Parameter $\varphi$ schließlich bezieht sich auf ein Zylinderkoordinatensystem des Auges und wird auf das Auge schauend entgegen dem Uhrzeigersinn gezählt, wie es in der Augenoptik üblich ist. Mit dem Wert $B_{BR}$ wird nun die Krümmung der modifizierten Hornhautvorderfläche 15* wie folgt eingestellt:

$$(2) \qquad R_{CV}{}^* = 1 \;/\; (\; (1/R_{CV}) + B_{BR} \;/\; (\; (n_c\text{-}1) \; (1 - d_{HS} \cdot B_{BR}))) + F$$

In Gleichung (2) bezeichnet $n_c$ die Brechkraft des Materials der Hornhaut. Der entsprechende Wert liegt üblicherweise bei 1,376; $d_{HS}$ bezeichnet den Abstand, in dem eine Brille mit der Brechkraft $B_{BR}$ vom Hornhautscheitel liegen muss, um die gewünschte Fehlsichtigkeitskorrektur mittels Brille zu erzeugen; $B_{BR}$ bezeichnet die zuvor erwähnte Brechkraft der Brille gemäß Gleichung (1). Die Angabe für die Brechkraft $B_{BR}$ kann auch Fehlsichtigkeiten erfassen, die über eine normale sphärische oder zylindrische Korrektur hinausgehen. $B_{BR}$ (und damit automatisch auch Rcv*) haben dann zusätzliche Koordinatenabhängigkeiten.

Der Faktor F drückt die optische Wirkung der Dickenänderung der Hornhaut aus und kann in erster Näherung als konstanter Faktor angesehen werden. Für eine hochgenaue Korrektur kann der Faktor gemäß folgender Gleichung errechnet werden:

$$(3) \qquad F = (1 - 1/n_c) \cdot (d_C{}^* - d_C).$$

dc bzw. dc* ist dabei die Hornhautdicke vor bzw. nach der optischen Korrektur. Für eine genaue Bestimmung erfolgt eine Berechnung von Rcv* iterativ, indem bei der i-ten Berechnung aus der Differenz (Rcv* - Rcv) auf die Größe (dc* - dc) geschlossen wird und das entsprechende daraus erhaltene Ergebnis für die Dickenänderung bei der (i+1)-ten Berechnung angewendet wird. Dies kann man so lange durchführen, bis ein Abbruchkriterium erfüllt wird, beispielsweise wenn die Differenz des Ergebnisses für die Dickenänderung bei zwei aufeinanderfolgenden Iterationsschritten unter einer entsprechend festgelegten Grenze liegt. Diese Grenze kann beispielsweise über eine konstante Differenz festgelegt werden, die einer für die Behandlung angemessene Genauigkeit der Refraktionskorrektur entspricht.

Vernachlässigt man die Dickenänderung der Augenhornhaut, was für ein vereinfachtes Verfahren durchaus zulässig ist, kann F in Gleichung (2) für eine vereinfachte Berechnung auch gleich Null gesetzt, also vernachlässigt und weggelassen werden. Man erhält überraschenderweise folgende einfache Gleichung für die Brechkraft der modifizierten Hornhaut 5*:

$$B_{CV}{}^* = B_{CV} + B_{BR} \;/\; (1 - B_{BR} \cdot d_{HS})$$

Aus dieser Gleichung ergibt sich für den Fachmann auf einfache Art und Weise mittels der Gleichung Bcv* = (n-1) / Rcv* der Radius Rcv* der Hornhautvorderfläche 15*, der nach der Modifikation vorliegen muss, um die gewünschte Fehlsichtigkeitskorrektur zu erhalten, zu: Rcv* = 1 / ( (1/Rcv) + $B_{BR}$ / ( ($n_c$-1) (1 - $d_{HS} \cdot B_{BR}$)))

Für das Volumen, dessen Entfernung die obige Krümmungsänderung der Hornhautvorderfläche 15 bewirkt, wird nun die das Volumen isolierende Grenzfläche festgelegt. Dabei ist vorzugsweise zu berücksichtigen, dass sich der Durchmesser des zu korrigierenden Bereichs und damit der Durchmesser des zu entnehmenden Volumens möglichst über die Pupillengröße bei dunkelangepasstem Auge erstrecken sollte.

In einer ersten Variante wird mittels dem Fachmann bekannter numerischer Methoden eine Freifläche definiert werden, die ein Volumen umschreibt, dessen Entfernung die Krümmungsänderung bewirkt. Dazu wird entlang der z-Achse die Dickenänderung ermittelt, die zur gewünschten Krümmungsmodifikation nötig ist. Daraus ergibt sich das Volumen als Funktion von r, $\varphi$ (in Zylinderkoordinaten) und daraus wiederum dessen Grenzfläche.

Eine einfache analytische Rechnung liefert die folgende zweite Variante, bei der die Grenzfläche des Volumens durch zwei Teilflächen aufgebaut wird, eine zur Hornhautoberfläche 15 hin gelegene anteriore Teilfläche und eine gegenüberliegende posteriore Teilfläche. Die entsprechenden Verhältnisse zeigt Figur 5. Das Volumen 18 ist zur Hornhautvorderfläche 15 hin durch eine anteriore Schnittfläche 19 begrenzt, die in konstantem Abstand $d_F$ unter der Hornhautvorderfläche 15 liegt. Diese anteriore Schnittfläche 19 wird in Analogie zur Laserkeratomen auch als Flap-Fläche 19 bezeichnet, da sie dort dazu dient, in Kombination mit einem Öffnungsschnitt zum Rand hin die Augenhornhaut 5 eine Lamelle in Form eines "Flap" von der darunterliegenden Hornhaut 5 abheben zu können. Diese Art der Entnahme des zuvor isolierten Volumens 18 ist natürlich auch hier möglich.

Die anteriore Schnittfläche 19 hat einen Krümmungsverlauf, der um $d_F$ unter der Hornhautvorderfläche 15 liegt. Ist diese sphärisch, ist kann für die Flap-Fläche 19 ein Krümmungsradius angegeben werden, der um $d_F$ geringer ist als der Krümmungsradius Rcv. Wie später für bevorzugte Varianten beschrieben wird, kann bei der Erzeugung der Schnittfläche 19 durch ein Kontaktglas dafür gesorgt werden, dass die Hornhautvorderfläche 15 zum Zeitpunkt der Schnittflächenerzeugung sphärisch ist, so dass das Muster der Zielpunkte eine sphärische Schnittfläche bewirkt. Die Relaxation des Auges 3 nach Abnahme des Kontaktglases mag dann zwar zu einer nicht-sphärischen Schnittfläche 19 führen, sie hat aber dennoch konstanten Abstand zur Hornhautvorderfläche 15 bzw. 15*. Dies wird später noch erläutert.

Posterior ist das Volumen 18, das aus der Hornhaut 5 entfernt werden soll, durch eine posteriore Schnittfläche 20 begrenzt, die schon grundsätzlich nicht zur Hornhautvorderfläche 15 in konstantem Abstand sein kann. Die posteriore Schnittfläche 20 wird deshalb so ausgebildet sein, dass das Volumen 18 in Form eines Lentikels vorliegt, weshalb die posteriore Schnittfläche 20 auch als Lentikel-Fläche 20 bezeichnet wird. In Figur 5 ist sie exemplarisch als ebenfalls sphärische Fläche mit einem Krümmungsradius $R_L$ eingezeichnet, wobei natürlich das Zentrum dieser Krümmung nicht mit dem Krümmungszentrum der in Figur 5 ebenfalls sphärischen Hornhautvorderfläche 15 zusammenfällt.

Figur 6 zeigt die Verhältnisse nach Entfernung des Volumens 18. Der Radius der modifizierten Hornhautvorderfläche 15* beträgt nun Rcv* und kann beispielsweise gemäß den zuvor beschriebenen Gleichungen berechnet werden. Die Dicke $d_L$ des entnommenen Volumens 18 ist dabei maßgeblich für die Radiusänderung, wie Figur 7 verdeutlicht. In dieser Figur sind als weitere Größen noch die Höhe $h_F$ der durch die anteriore Schnittfläche 19 definierten Kugelkappe, die Höhe $h_L$ der durch die posteriore Schnittfläche 20 definierten Kugelkappe sowie die Dicke $d_L$ des zu entfernenden Volumens 18 eingezeichnet.

Die posteriore Schnittfläche 20 legt aufgrund des konstanten Abstandes zwischen Hornhautvorderfläche 15 und anteriorer Schnittfläche 19 den Krümmungsverlauf der Hornhautvorderfläche 15* nach Entfernung des Volumens 18 fest. Somit wird die posteriore Schnittfläche 20 z.B. bei einer zylindrische Parameter berücksichtigenden Fehlsichtigkeitskorrektur einen winkelabhängigen Krümmungsradius haben. Für die in Figur 7 gezeigte Lentikel-Fläche 20 gilt allgemein:

$$R_L(\varphi) = R_{CV}{}^*(\varphi) - d_F \, ,$$

bzw. in Zylinderkoordinaten (z, r, $\varphi$)

$$z_L(r, \ \varphi) = R_L(\varphi) - (R_L{}^2(\varphi) - r^2)^{1/2} \ + d_L + d_F.$$

Ohne Berücksichtigung eines Astigmatismus entfällt die Abhängigkeit von $\varphi$ und die Lentikel-Fläche 20 ist sphärisch. Die Lentikel-Fläche 20 besitzt aber, geht man vom Bedarf für eine zylindrische Fehlsichtigkeitskorrektur aus, in der Regel auf verschiedenen Achsen unterschiedliche Krümmungsradien, wobei diese natürlich meist den gleichen Scheitelpunkt haben.

Damit wird weiter automatisch deutlich, dass im Fall einer Zylinderkorrektur die theoretische Schnittlinie zwischen Flap-Fläche 19 und Lentikel-Fläche 20 nicht in einer Ebene, d.h. bei konstanten z-Koordinaten liegt. Der kleinste Krümmungsradius der Lentikel-Fläche 20 liegt bei $\varphi = \theta + \pi/2$, der größte natürlich auf der Achse $\theta$ der zylindrischen Fehlsichtigkeit, d.h. bei $\varphi = \theta$. Bei einer Übersichtigkeitskorrektur fallen anders bei der Darstellung der Figur 7 der Scheitelpunkt von Flap-Fläche 19 und Lentikel-Fläche 20 zusammen und die Lentikel-Fläche 20 ist stärker gekrümmt, als die Flap-Fläche 19. Die Dicke $d_L$ des Lentikels ergibt sich als Randdicke.

Das als Lentikel aufzufassende Volumen 18 hat bei $\varphi = \theta = \pi/2$ die geringste Randdicke, da sich dort Lentikel-Fläche 20 und Flap-Fläche 19 schneiden. Bei allen anderen Werten für $\varphi$ ist eine endliche Randdicke gegeben, wenn eine gegebene z-Koordinate als untere Grenze der Lentikel-Fläche 20 angesetzt wird.

Alternativ kann neben der Flap-Fläche 20 und der Lentikel-Fläche 19 zusätzliche Randfläche vorgesehen werden, welche das Volumen 18 im Schnittbereich von Flap-Fläche 20 und der Lentikel-Fläche 19 umrandet bzw. diese Flächen dort verbindet, wo sie bei einer gegebenen z-Koordinate nicht zusammenlaufen. Der Schnitt dieser Randfläche wird ebenfalls mit dem gepulsten Laserstrahl ausgeführt. Die Randfläche kann beispielsweise eine zylindrische Form haben, die jedoch auch eine elliptische Form (in der Aufsicht) oder auch eine konische Form (in der Seitenansicht haben kann).

Die in den Figuren gezeigte Ausbildung des Volumens 18 als durch eine anteriore Schnittfläche 19 mit konstantem Abstand zur Hornhautvorderfläche 15 sowie eine posteriore Schnittfläche 20 begrenzt, ist nur eine Variante zur Begrenzung des Volumens 18. Sie hat jedoch den Vorteil, dass die optische Korrektur wesentlich nur durch eine Fläche (die Lentikelfläche 20) festgelegt wird, so dass die analytische Beschreibung der anderen Teilfläche der Grenzfläche einfach ist.

Weiter sind optimale Sicherheitsmargen hinsichtlich des Abstandes des Volumens zur Hornhautvorderfläche 15 und Hornhautrückfläche 16 bietet. Die Restdicke $d_F$ zwischen anteriorer Schnittfläche 19 und Hornhautvorderfläche 15 kann konstant auf einen Wert von beispielsweise 50 bis 200 μm eingestellt werden. Insbesondere kann sie so gewählt sein, dass das schmerzempfindliche Epithel in der Lamelle verbleibt, die durch die Flap-Fläche 19 unter der Hornhautvorderfläche 15 gebildet ist. Auch steht die Ausbildung der sphärischen Flap-Fläche 19 in Kontinuität mit bisherigen Keratometerschnitten, was für die Akzeptanz der Methode vorteilhaft ist.

Nach Erzeugen der Schnittflächen 19 und 20 wird dann das derart isolierte Volumen 18 aus der Hornhaut 5 entfernt. Dies ist schematisch in Figur 8 dargestellt, die zudem verdeutlicht, dass die Schnittflächen 19 und 20 durch Einwirkung des in einem Fokuskegel 21 einfallenden Behandlungslaserstrahls erzeugt werden, beispielsweise durch Aneinanderreihung von Plasmablasen, so dass in einer bevorzugten Ausführungsform die Flap-Schnittfläche 19 und die Lentikel-Schnittfläche 20 durch geeignete dreidimensionale Verstellung der Fokuslage der gepulsten Laserstrahlung 2 erzeugt werden.

Erzeugt man sowohl die Lentikel-Fläche 20 als auch die Flap-Fläche 19 mittels gepulster Laserstrahlung, ist es zweckmäßig, die Lentikel-Fläche 20 vor der Flap-Fläche 19 auszubilden, da das optische Ergebnis bei der Lentikel-Fläche 20 besser (wenn nicht überhaupt erst zu erreichen) ist, wenn oberhalb der Lentikel-Fläche 20 noch keine Veränderung der Hornhaut 5 eintrat.

Das Entfernen des durch die gepulste Laserstrahlung isolierten Volumens 18 kann, wie in Figur 8 angedeutet, durch einen Randschnitt 22 erreicht werden, der es erlaubt, das Volumen 18 in Richtung eines in Figur 8 eingezeichneten Pfeiles 23 herauszuziehen. Alternativ kann der Randschnitt 22 aber so ausgebildet werden, dass er die anteriore Schnittfläche 19, d. h. die Flap-Fläche 19, in Form eines Ringes mit der Hornhautvorderfläche 15 verbindet, wobei der Randschnitt allerdings nicht vollständig um einen Winkel von 360° umläuft. Die derart isolierte Lamelle bleibt in einem schmale Bereich mit dem übrigen Gewebe der Hornhaut 5 in Verbindung. Diese Verbindungsbrücke dient dann als Gelenk, um die ansonsten isolierte Lamelle von der Hornhaut 5 abzuklappen und das dadurch zugängige, bereits isolierte Volumen 18 vom Rest der Augenhornhaut 5 abnehmen zu können. Die Lage der Verbindungsbrücke ist bei Erzeugung der Steuerdaten bzw. der Zielpunkte vorgebbar. Das beschriebene Vorgehen bzw. Gerät realisiert also unter diesem Gesichtspunkt die Isolierung des Volumens 19 innerhalb der Hornhaut 5 und das Erzeugen einer mit der restlichen Augenhornhaut über eine Gewebebrücke verbundenen Lamelle als Deckel über dem Volumen. Der Deckel kann abgeklappt und das Volumen 18 entnommen werden.

Für die Erzeugung der Schnittflächen 19 und 20 können die Zielpunkte nun auf verschiedenste Art und Weise angeordnet werden. Im Stand der Technik ist beispielsweise in der WO 2005/011546 zur Erzeugung von Schnittflächen in der Augenhornhaut beschrieben, dass spezielle Spiralen eingesetzt werden können, die beispielsweise um eine im Wesentlichen senkrecht zur optischen Achse (z-Achse) liegende Hauptachse in Art einer Schraubenlinie verlaufen. Auch ist die Verwendung eines Scanmusters bekannt, das die Zielpunkte zeilenweise anordnet (vgl. WO 2005/011545). Diese Möglichkeiten können selbstverständlich zur Erzeugung der oben definierten Schnittflächen verwendet und mit den nachfolgend erläuterten Transformationen werden.

Die Verstellung der Lage des Fokus in der Augenhornhaut erfolgt mittels der in Figur 3 schematisch dargestellten dreidimensionalen Ablenkeinrichtung, die zur Verstellung des Fokus in z-Richtung die Verschiebung von Linsen oder anderer optisch wirksamer Elemente einsetzt. Nun ist die Verstellung von Linsen o.ä. in der Regel nicht so schnell möglich, wie die Verschwenkung von Spiegeln, wie sie in der Regel im xy-Scanner Einsatz finden. Meist ist deshalb die Verstellgeschwindigkeit des z-Scanners begrenzend für die Geschwindigkeit, mit der die Schnittflächen in der Augenhornhaut erzeugt werden können. Zur möglichst schnellen Erzeugung der Schnittflächen 18 und 19 wird deshalb in einer bevorzugten Ausführungsform der Fokus jeweils entlang einer spiralförmigen Bahn geführt, wobei je eine Spirale in der räumlich gekrümmten Schnittfläche liegt. Während des Schreibens der Spirale wird also der z-Scanner so verstellt, dass die Arme der Spirale der räumlich gekrümmten Schnittfläche folgen.

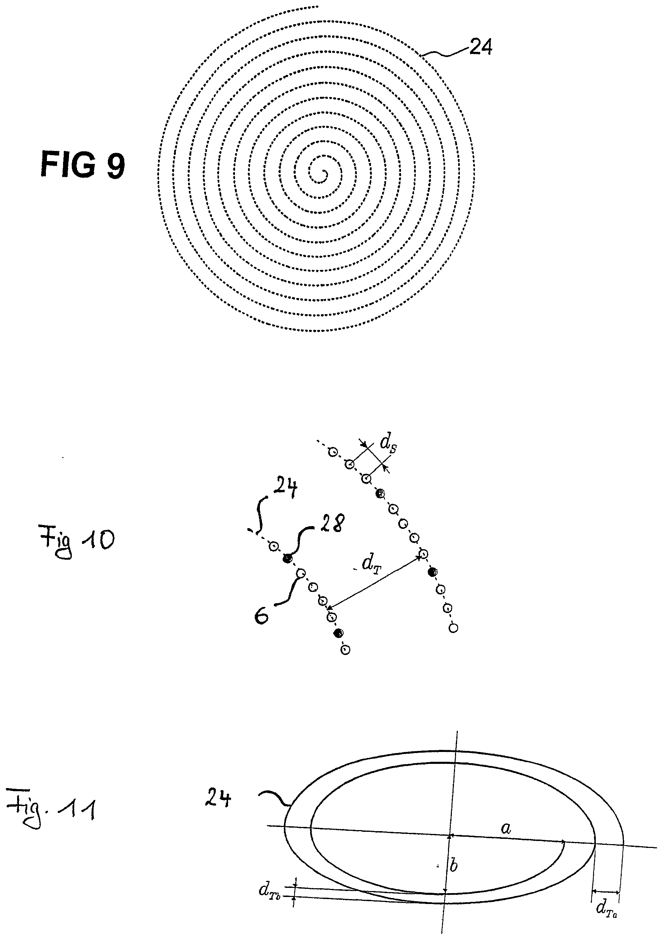

Figur 9 zeigt exemplarisch eine Bahnkurve 24 als Spirale, die in der gezeigten Darstellung als Kreisspirale ausgebildet ist. Der Radius der dargestellten ebenen Spirale nimmt in Kreiskoordinaten mit steigendem Drehwinkel φ zu, so dass gilt:

$$(4) \qquad r(\varphi) = \varphi \cdot d_T / (2\pi)$$

In dieser Gleichung bezeichnet $d_T$ den Abstand der Spiralarme; er ist in Figur 10 dargestellt, die einen vergrößerten Ausschnitt der Figur 9 zeigt. Der Abstand der einzelnen Spots 6, auf die gepulste Laserstrahlung fokussiert wird und an denen durch einen Laserpuls beispielsweise eine Plasmablase erzeugt wird, ist in der Spirale konstant gleich ds, so dass für den Winkelabstand $\Delta\varphi$ der einzelnen Spots 6, an denen ein Laserpuls in das Gewebe eingebracht wird, gilt:

$$\Delta\varphi = d_S / r$$

Da, wie bereits erwähnt, die Lentikel-Fläche 20 in der Regel nichtsphärisch ist, ist die Bahnkurve 24, entlang der der Laserfokus verstellt wird, eine elliptische Spirale, für die natürlich kein konstanter Abstand der Spiralarme mehr gegeben ist. Entlang der Hauptachsen a und b kann aber ein jeweiliger Bahnabstand $d_{Tb}$ sowie $d_{Ta}$ definiert werden, wie Figur 11 zeigt.

In Figur 10 sind die Spots 6 dargestellt, um die Lage des Fokus für die einzelnen Laserpulse erkennen zu lassen. Tatsächlich weiten sich die Plasmablasen natürlich nach Einbringung des jeweiligen Laserpulses so weit auf, dass die Schnittfläche erzeugt wird und die Bahnkurve 24 ist in der Schnittfläche dann nicht mehr zu erkennen.

Zur Vorbereitung des chirurgischen Verfahrens muss nach der Definition der Schnittflächen 19 und 20 nun die Definition der Bahnkurven 24 erfolgen, mit denen die Schnittflächen erzeugt werden.

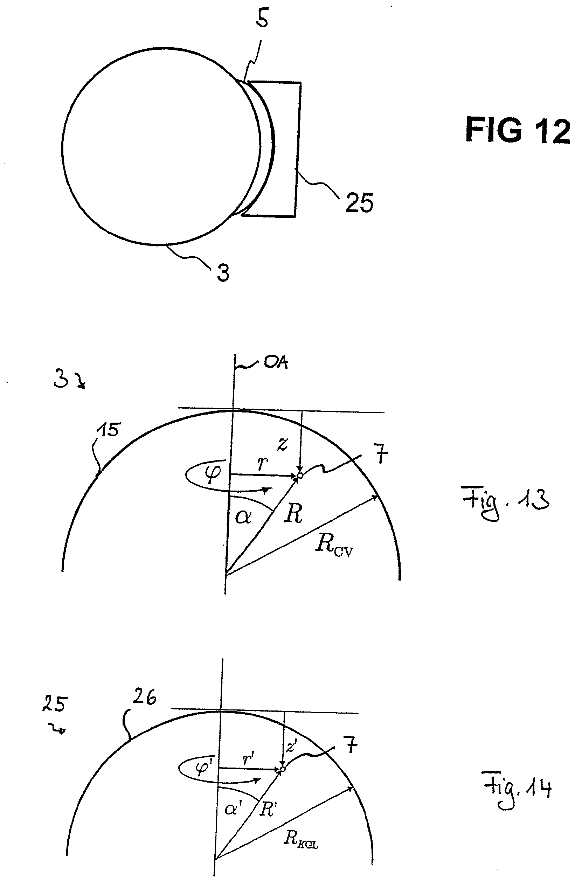

Bei der Bestimmung der Bahnkurven 24 ist natürlich zu berücksichtigen, dass letztendlich das Volumen 18 im Auge im Normalzustand definiert sein soll. Die Schnittflächen 19 und 20, wie sie bislang erläutert wurden, betreffen das natürliche Auge. Es ist nun aber zu berücksichtigen, dass das Behandlungsgerät 1 aus Gründen der Fixierung des Auges mit einem Kontaktglas 25 arbeitet, das wie in Figur 12 gezeigt ist, auf die Hornhautvorderfläche 15 der Augenhornhaut 5 aufgesetzt wird. Das Kontaktglas 25, das bereits Gegenstand mehrerer Patentpublikationen ist (exemplarisch sei beispielsweise auf die WO 2005/048895 A verwiesen), ist für die hier vorliegende Beschreibung des Behandlungsgerätes 1 bzw. der damit in Zusammenhang stehenden Verfahren zur Vorbereitung und/oder Durchführung des chirurgischen Eingriffes allerdings nur insoweit von Interesse, als es der Hornhautvorderfläche 15 zum einen eine definierte Krümmung verleiht und zum anderen die Augenhornhaut 5 gegenüber dem Behandlungsgerät 1 räumlich in einer vordefinierten Lage hält. Hinsichtlich der sphärischen Krümmung der Kontaktfläche des Kontaktglases 25 unterscheidet sich der hier beschriebene Ansatz jedoch deutlich von dem Ansatz, wie er beispielsweise in der WO 2003/002008 A beschrieben ist, der ein planes Kontaktglas verwendet, welches die Augenhornhaut flachdrückt.

Wird das Auge an das Kontaktglas 25 mit sphärischer Kontaktfläche angepresst, kommt es zu einer räumlichen Deformation des Auges. Da die Kornea regelmäßig nur tangential kompressibel ist, also bei einem solchen Anpressen ihre Dicke nicht ändert, entspricht das Anpressen einer Transformation vom Koordinatensystem des Auges, wie es in Figur 13 dargestellt ist, in das Koordinatensystem des Kontaktglases, das in Figur 14 gezeigt ist. Dieser Zusammenhang ist dem Fachmann aus der WO 2005/011547 A1 bekannt, deren Offenbarungsgehalt diesbezüglich vollumfänglich eingebunden sein soll. In den Figuren 13 und 14 bezeichnen mit einem Apostroph versehene Koordinaten die Koordinaten des auf das Kontaktglas 25 bzw. dessen dem Auge zugewandte Kontaktglasunterseite 26 bezogenen Größen.

Das Kontaktglas hat aber noch einen weiteren Vorteil. Durch das Anpressen an die sphärische Kontaktglasunterseite 26 ist automatisch auch die Hornhautvorderfläche 15 sphärisch. Die in konstantem Abstand unter der Hornhautvorderfläche 15 liegende anteriore Schnittfläche 19 ist damit bei angepresstem Kontaktglas ebenfalls sphärisch, was zu erheblich vereinfachter Ansteuerung führt. Es ist deshalb völlig unabhängig von anderen Merkmalen bevorzugt, ein Kontaktglas 25 mit sphärischer Kontaktglasunterseite 26 zu verwenden und das Volumen durch eine anteriore Schnittfläche 19 sowie eine posteriore Schnittfläche zu begrenzen, wobei für die anteriore Schnittfläche Zielpunkte vorgegeben sind/werden, die diese Schnittfläche als sphärische Fläche in konstantem Abstand $d_F$ unter der Hornhautvorderfläche 15 ausbilden. Für die posteriore Schnittfläche sind/werden Zielpunkte vorgegeben, die einen Krümmungsverlauf definieren, welcher bei relaxiertem Auge, also nach Abnehmen des Kontaktglases, bis auf den Abstand $d_F$ zur Hornhautvorderfläche dem zur Fehlsichtigkeitskorrektur gewünschten entsprechen. Analoges gilt für das Verfahren zur Definition der Zielpunkte bzw. das Operationsverfahren.

Die Darstellungen in den Figuren 13 und 14 zeigen die Koordinatentransformation, die am Auge durch das Aufsetzten bzw. Abnehmen des Kontaktglases auftritt. Sie enthalten sowohl Kugelkoordinaten $(R, \alpha, \varphi)$ bezogen auf den Ursprung der gekrümmten Fläche (Hornhautvorderfläche 15 bzw. Kontaktglasunterseite 26) als auch Zylinderkoordinaten $(r, z, \varphi)$ bezogen auf den durch den Durchtrittspunkt der optischen Achse OA definierten Scheitelpunkt der Hornhautvorderfläche 15 bzw. der Kontaktglasunterseite 26.

Bei der Koordinatentransformation vom auf das Auge bezogenen Koordinatensystem, wie es in Figur 13 dargestellt ist, in das auf das Kontaktglas bezogene System gemäß Figur 14 bleiben die Bogenlänge, d.h. $\alpha \cdot R$, die radiale Tiefe $(R_{cv} - R)$ sowie der Winkel $\varphi$ erhalten. Die Transformation der für das natürliche Auge, d.h. im Koordinatensystem der Figur 13, zugrunde gelegten Formen der Schnittflächen 19 und 20 ist somit ein wichtiger Schritt bei der Berechnung der Ansteuergrößen für die dreidimensionale Fokusverstelleinrichtung. Sie verläuft grundsätzlich anders als bei einem ebenen Kontaktglas, in dem z.B. die Flap-Fläche 19 zu einer Ebene entartet. Im Wesentlichen ist nur die Form für die Schnittfläche 20 zu transformieren, da die Schnittfläche 19 lediglich in konstantem Abstand $d_F$ zur Hornhautvorderfläche 15 auszubilden ist. Die Schnittfläche 19 ist also im transformierten System eine Sphäre mit einem gegenüber der Kontaktglasunterseite um $d_F$ reduzierten Krümmungsradius $R_F$.

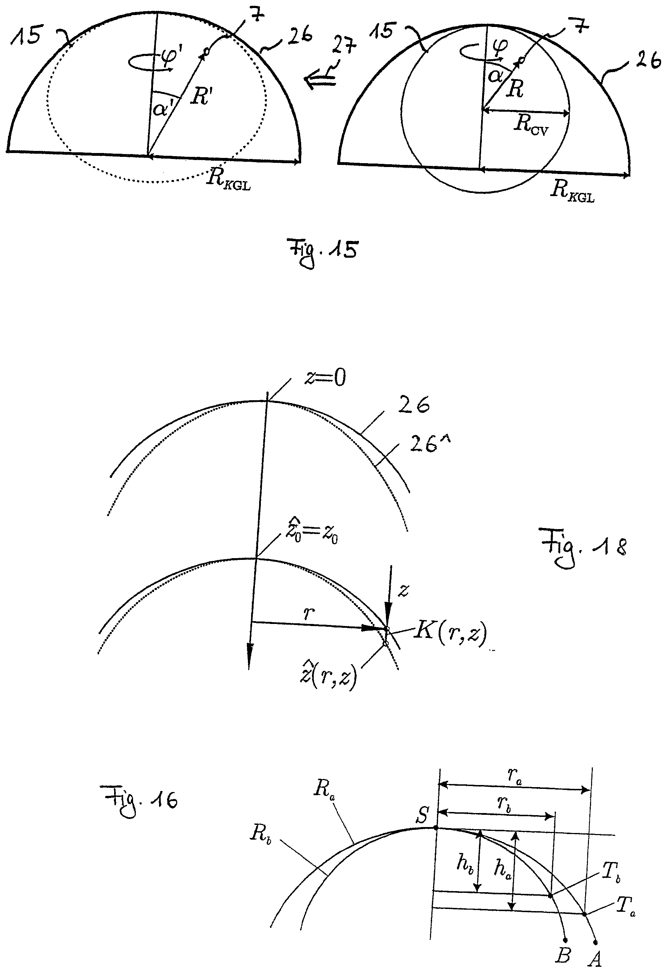

Das Anpressen der Hornhaut 5 des Auges 3 an die sphärisch gekrümmte Kontaktglasunterseite 26 ist in Figur 15 veranschaulicht. Dort zeigt die rechte Darstellung schematisch den Zustand, wenn die Kontaktglasunterseite 26 nur am Scheitelpunkt in Kontakt mit der Hornhautvorderfläche 15 ist. Zur Verdeutlichung der geometrischen Beziehungen ist die Hornhautvorderfläche 15 schematisch in Figur 15 als Kreis eingezeichnet, obschon natürlich die sphärische Krümmung nur in einem kleineren Kreisabschnitt vorliegt. Das Anpressen des Kontaktglases 25 auf die Hornhaut 5 bewirkt den durch Pfeil 27 symbolisierten Übergang zum Zustand der linken Seite der Figur 15. Das Abnehmen des Kontaktglases 25 bewirkt eine Relaxation des Auges 3 entgegen der Richtung des Pfeiles 27

Aufgrund der geschilderten Rahmenbedingungen transformieren sich für jeden Punkt in der Augenhornhaut 5 die Koordinaten von dem in Figur 13 dargestellten System in das System der Figur 14. Dies wird nun bei der Wahl der Ansteuerwerte für die Fokusverstellung dahingehend zugrunde gelegt, dass die Schnittflächen 19 und 20 im transformierten Kontaktglassystem zu beschreiben sind, da sie nur dann nach Abnehmen des Kontaktglases 26, d.h. nach RückTransformation in das natürliche Koordinatensystem des Auges, die gewünschten Formen haben. Da das Anlegen der Hornhautvorderfläche 15 in der Regel durch Ansaugen mittels Unterdruck bewirkt wird, wird die geschilderte Transformation nachfolgend auch als Ansaugtransformation bezeichnet.

Zum Schneiden der Flap-Fläche 19, die wie erwähnt sphärisch ist, wird nun folgende Geschwindigkeit der Verstellung des z-Scanners, d.h. folgende Vorschubgeschwindigkeit in z-Richtung eingestellt:

$$(5) \qquad v_z(t) = d_S \bullet f_L \bullet d_T / (2\pi \bullet (R_F^2 - t \bullet d_S \bullet f_L \bullet d_T / \pi))^{1/2},$$

wobei $f_L$ die Frequenz der Laserpulse der Laserstrahlung 2 ist. Gleichung (5) setzt voraus, dass die z-Geschwindigkeit vz frei eingestellt und kontinuierlich verändert werden kann.

Möchte man eine Sphäre mit einer Geschwindigkeit vz schreiben, die aus einer Gruppe diskreter Geschwindigkeiten gewählt ist, was in der Regel dann der Fall ist, wenn der z-Scanner mittels eines Schrittmotors angetrieben ist, erhält man als Zeitabhängigkeit der Radialfunktion r(t):

$$(6) \qquad r(t) = [d_S \bullet f_L \bullet d_T \bullet t/\pi - (d_S \bullet f_L \bullet d_T \bullet t)^2/(2\pi \bullet R_F)^2]^{1/2}$$

sowie für die Winkelfunktion

$$(7) \qquad \varphi(t) = [4\pi \bullet d_S \bullet f_L \bullet t/d_T - (d_S \bullet f_L \bullet t)^2/R^2]^{1/2}.$$

Die $t^2$-Terme unter der Wurzel der Radial- wie der Winkelfunktion zeigen, dass keine ideale archimedrische Spirale mehr geschrieben wird, die Bahn- und Spotblasen-Abstände variieren also zugunsten der nur in Stufen veränderlichen z-Geschwindigkeit.

Wünscht man bei der Fokusverstellung einen konstanten z-Vorschub, ergibt sich nicht, wie mit der Geschwindigkeit gemäß Gleichung (4) eine Sphäre, sondern ein Paraboloid, und es gilt:

$$(8) \qquad z(r) = [v_z/(d_S \bullet d_r)][r^2 \bullet \pi/f_L]$$

Erwähnter Weise kann in manchen Behandlungsgeräten 1 die Geschwindigkeit, mit der der z-Scanner den Fokus in z-Richtung verschiebt, nur innerhalb eines Satzes diskreter Geschwindigkeiten verstellt werden. Möchte man dann eine bestimmte Parabel mit einer gegebenen Geschwindigkeit vz beschreiben, muss das Produkt $d_S \bullet d_T$ entsprechend gewählt werden, so dass die erste eckige Klammer der Gleichung (8) den gewünschten Wert einnimmt. Der Abstand der Bahnen, definiert durch $d_T$, sowie der Spotabstand entlang der Bahn beschrieben durch ds, sind also geeignet zu variieren, um eine bestimmte Parabel mit gegebenen $v_z$ zu schreiben.

Jede der Gleichungen (5), (6)/(7) und (8) kann bei der Ermittlung der Zielpunkte und damit de Ansteuerung der Fokusverstellung verwendet werden, wobei dann natürlich die entsprechende Spiralform/Flächenform zugrunde zu legen ist. Wenn nachfolgend davon gesprochen wird, dass die Gleichungen bei der Ansteuerung verwendet werden, ist darunter insbesondere zu verstehen, dass mittels der Gleichungen die Zielpunkte ermittelt werden, die kann z.B. durch Auswerten der Funktionsgleichungen zu äquidistanten Zeitpunkten geschehen. Die Geschwindigkeitsgleichungen werden in einer Variante dazu verwendet, sicherzustellen, dass die ermittelten Zielpunkte keine Verstellgeschwindigkeiten bedingen, die von der Fokusverstelleinrichtung gar nicht realisierbar sind.

Für die eingangs erwähnten und wie beschrieben ermittelten Formen der Flächen 19 und 20 wird nun eine Spirale in die jeweilige Fläche gelegt. Sie Spirale wird durch eine Ansteuerung der beschriebenen Art geschrieben. Die Berechnung der z-Geschwindigkeit sowie der r- und φ-Geschwindigkeit berücksichtigt dabei, welche Flächenform die Fläche 19 bzw. 20 hat.

Die Lentikel-Fläche 20 ist sphärisch, wenn keine Zylinderkorrektur vorgenommen werden soll. Man verwendet deshalb die Ansteuerung gemäß Gleichungen (4)/(5) oder (6)/(7) um diese Sphäre zu erzeugen. Allerdings kann eine Sphäre bekanntermaßen auch durch ein Paraboloid approximiert werden. Es ist deshalb in einer Variante vorgesehen, die eine Sphäre auf dem Fachmann bekannter Weise durch ein Paraboloid anzunähern und die Ansteuerung gemäß Gleichung (8) vorzunehmen.

Durch die Ansaugtransformation gemäß Figur 15 verändert sich die Geometrie der Lentikel-Fläche 20. Die Lentikel-Fläche 20 muss im Koordinatensystem des Kontaktglases 25 den Krümmungsverlauf der korrigierten Hornhautvorderfläche 15* aufweisen. Sie kann nicht auf einen Krümmungsmittelpunkt bezogen werden, der mit dem Krümmungsmittelpunkt des Kontaktglases zusammenfällt. Die gemäß Gleichung (2) definierte Krümmung wird also bezüglich der Ansaugtransformation umgerechnet.

Der in Gleichung (2) definierte Krümmungsradius ist natürlich eine Funktion von φ. Wie bereits erwähnt und in der Augenoptik üblich, können zwei Krümmungsradien $r_a$ bzw. $r_b$ angegeben werden: einer auf der Achse θ der zylindrischen Fehlsichtigkeit und einer für eine Achse rechtwinklig dazu. Rechentechnisch ist es besonders günstig, die sich somit im allgemeinen Fall einstellende toroidale Krümmung durch eine Parabel zu approximieren, so dass die Lentikel-Fläche 20 durch ein Paraboloid angenähert wird. Dies geschieht vorzugsweise vor der Anpresstransformation kann aber auch danach durchgeführt werden.

Die Annäherung erfolgt dadurch, dass man für die zwei Krümmungsradien jeweils eine Parabel sucht, die sowohl durch den Scheitelpunkt der Lentikel-Fläche 20 als auch durch einen möglichst am Rand gelegenen Punkt läuft. Die entsprechenden Verhältnisse im Koordinatensystem des Auges zeigt Figur 16. Diese Figur zeigt die sphärische Lentikel-Fläche 20 vor der Anpresstransformation. In der Figur sind die Schnitte durch die im verallgemeinerten Fall toroidale Lentikel-Fläche 20 entlang der zwei Halbachsen a und b übereinandergelegt. Die entsprechenden Kurven sind mit A bzw. B bezeichnet und sind kreisförmig mit einem Krümmungsradius $r_a$ bzw. $r_b$. Auf jeder Kurve ist ein Randpunkt T in Zylinderkoordinaten durch den entsprechenden Radius r sowie die Höhe h beschrieben, wobei diese Parameter auf den Scheitelpunkt S bezogen sind, der für die zwei Halbachsenschnitte identisch ist. Der Punkt $T_a$ ist also durch den Radius $r_a$ sowie die Höhe $h_a$ gekennzeichnet. Analoges gilt für $T_b$.

Es wird nun eine Parabel gesucht, für die gilt $h = k - r^2$. Die dadurch erhaltenen Parabelparameter $h_a$ für die Parabel entlang der großen Halbachse a sowie $k_b$ für die Parabel entlang der kleinen Halbachse b definieren das Paraboloid, das dann unter Verstellung des Fokuspunktes in z-Richtung geschrieben wird, beispielsweise mittels eines konstanten z-Vorschubes (vgl. Gleichung (7)) bzw. die mit einer Auswahl der z-Geschwindigkeit aus einem Satz diskreter Geschwindigkeiten gewählt wird (Modifikation zur Gleichung (7)). Die in Figur 16 dargestellten Schnitte der toriodalen Lentikel-Fläche 20 entlang der kleinen Hauptachse b sowie der großen Hauptachse a beziehen sich auf die Darstellung im Koordinatensystem des Auges.

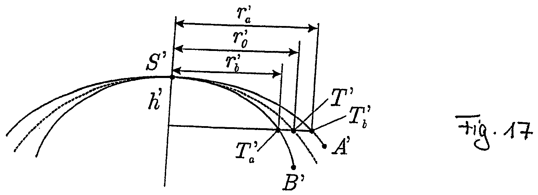

Wenn die Approximation durch Parabelgleichungen nach der Anpresstransformation durchgeführt wurde, treten dort natürlich die transformierten Werte auf. Man kann die explizite Berechnung von transformierten Werten an dieser Stelle vermeiden, wenn die Approximation zuerst erfolgt und die dabei gefundenen Parabelparameter der Anpresstransformation in das Koordinatensystem des Kontaktglases unterworfen werden, wonach dann die in Figur 17 dargestellten Verhältnisse vorliegen.

Die Spezifikation der Parabelparameter lautet im Koordinatensystem des Auges gemäß Figur 16 wie folgt:

$$(9) \qquad k_a = (\, z(T_a) - z(S) \,) \,/\, r(T_a)^2,$$

$$(10) \qquad k_b = (\, z(T_b) - z(S) \,) \,/\, r(T_b)^2.$$

In den Gleichungen (9) und (10) bezeichnet z(S) die z-Koordinate des Punktes S. Legt man den Koordinatensystemursprung, wie in den bisherigen Figuren, in den Scheitelpunkt, ist die z-Koordinate Null. Die Koordinate $z(T_a)$ bzw. $z(T_b)$ sowie $r(T_a)$ bzw. $r(T_b)$ sind die z- bzw. r-Koordinaten des entsprechenden Punktes $T_a$ bzw. $T_b$ im zylindrischen Koordinatensystem.

Werden die Parabelparameter $k_a$ bzw. $k_b$ nicht im Koordinatensystem des Auges gemäß Figur 16, sondern im Koordinatensystem des Kontaktglases gemäß Figur 17 benötigt, treten anstelle der Punkte S, $T_a$ und $T_a$ dann die in Figur 17 eingezeichneten transformierten Punkte S', $T_a$', $T_b$'.

Um die Lentikel-Fläche 20 nun im angepressten Auge 3 durch eine (dann in der Regel elliptische) Spirale mit den Hauptachsen $r_a$' und $r_b$' darzustellen, wird die Spirale aus einer Kreisspirale mit Radius $r_0$' durch Streckung in Richtung φ = θ und Stauchung in Richtung φ = θ + π/2 konstruiert. Durch die gleichzeitige Stauchung und Streckung bleibt der mittlere Bahn- bzw. Spotabstand erhalten. Würde man nur in eine Richtung stauchen oder strecken, würde der mittlere Abstand verändert.

Die eigentlichen Radien lassen sich aus dem Radius $r_0$ der Kreisspirale, der in Figur 17 gestrichelt eingezeichnet ist, mit Hilfe der Elliptizität wie folgt berechnet:

$$e' = r_a'/r_b' = (k_B'/k_a')^{1/2}.$$

Die Elliptizität e' ist dabei die Elliptizität der transformierten toriodalen Lentikel-Fläche 20. Die Parameter $k_b'$ sowie $k_a'$ sind durch die Gleichungen (11) und (12), jeweils für die transformierten Punkte S', $T_a'$ und $T_b'$ gegeben. Die Parabelparameter ergeben sich daraus, dass hier die Kreisspirale mit dem Radius $r_0$, aus dem die Lentikel-Fläche 20 konstruiert ist, ein arithmetisches oder geometrisches Mittel der Krümmungen der großen bzw. kleinen Halbachse des Paraboloid sein soll. Wegen $r_0' = (r_a' \cdot r_b')^{1/2}$ erhält man für den Parabelparameter $k = (k_a \cdot k_b)^{1/2}$ sowie die Halbachsen der Ellipse: $r_a' = r_0' \cdot (e')^{1/2}$ und $r_b' = r_0' \cdot (e')^{-1/2}$.

An dieser Stelle der Ermittlung der Zielpunkte liegen nun zwei Bahnkurven 24 vor, die durch Funktionsgleichungen beschrieben sind. Das Muster der Zielpunkte wird durch Auswertung der Funktionsgleichungen ermittelt.

Allerdings bleibt noch zu berücksichtigen, dass die Fokussierung des Laserstrahls in den Fokus 7 einem Fokuslagenfehler unterliegt. Dieser Fokuslagenfehler ist Eigenschaft des optischen Systems, d. h. beruht auf der verwendeten optischen Realisierung. Er ist im Wesentlichen durch das optische Design bestimmt. Aufgrund endlicher Fertigungsgenauigkeiten im Rahmen der erlaubten Toleranz ist der Fokuslagenfehler darüber hinaus geräteindividuell. Er wird deshalb zweckmäßigerweise für jedes Gerät eigenständig bestimmt.

Die Berücksichtigung des Fokuslagenfehlers erfolgt durch eine in der Regel nichtlineare Transformation (nachfolgend auch als NL-Transformation bezeichnet). Es ist deshalb nicht möglich, die NL-Transformation durch Modifikation der Bahnkurvenparameter auszuführen. In einer bevorzugten Ausführungsform wird der Fokuslagenfehler durch eine Korrekturtabelle oder eine Korrekturfunktion ausgedrückt. Sie stammt aus einer Vermessung der Optik des Behandlungsgerätes 1. Die Vermessung kann gerätetypbezogen oder geräteindividuell geschehen. Die Korrekturfunktion kann aus einer Interpolation der Messresultate z. B. mittels Polynomen oder Splines gewonnen sein. Meist ist der Fokuslagenfehler rotationssymmetrisch bezogen auf die optische Achse. Er hängt dann in Zylinderkoordinaten nur von r und z ab.

Die zuvor mittels der Bahnkurven errechneten Punkte werden in der NL-Transformation so vorverzerrt, dass sie nach der Einbringung der Laserspots mit dem optischen System, das den Fokuslagenfehler aufweist, genau an der gewünschten Stelle liegen. Die Anwendung der vorverzerrten Koordinaten kompensiert also den im optischen System auftretende Fokuslagenfehler.

Die NL-Transformation geht von der Überlegung aus, dass man zu jedem Punkt mit den Koordinaten (z, r) eine um $z_0$ verschobene Kontaktglassphäre findet, auf der dieser Punkt liegt. Der Scheitelpunkt dieser Sphäre ist dann gerade bei $z_0(z, r) = z - z_{KGL}(r)$. Die Wirkung der Vorverzerrung zur Kompensation des Fokuslagenfehlers ist in Figur 18 veranschaulicht. Sie zeigt die Kontaktglasunterseite 26, die im vorliegenden Beispiel sphärisch ist, aber auch eine andere Form haben kann. Aufgrund der Vorverzerrung wird sie zu einer transformierten Kontaktglassphäre 26^. Die die Vorverzerrung des Fokuslagenfehlers berücksichtigenden Parameter sind in Figur 18 durch ein angefügtes Dach "^" symbolisiert. Für einen transformierten Achsenpunkt in der Kornea gilt dann $z_0^{\wedge} = z_0$. Dies trägt der Tatsache Rechnung, dass der Fokuslagenfehler zwar meist rotationssymmetrisch ist, allerdings auch eine Verschiebung in z-Richtung zur Folge hat.

Zur Vorverzerrung werden die berechneten Bahnkurven in individuelle Zielpunkt-Koordinaten für die Spots umgesetzt, welche dann in der in Figur 18 durch K(r, z) symbolisierten Korrekturtransformation, also der NL-Transformation, verschoben werden. Ist der Fokuslagenfehler als Funktion K angeben, muss die Koordinate eines jeden Zielpunktes lediglich mit der Funktion ausgewertet werden, um die Verschiebung bzw. die transformierte Koordinate zu erhalten.

Im Ergebnis liegt dann für die Flächen 19 und 20 jeweils ein Satz Zielpunkt-Koordinaten vor, entlang der der Fokus geführt wird. Durch die NL-Transformation und die Anpresstransformation liegen die Koordinaten bezogen auf das natürliche, also freie Auge genau in den gewünschten anterioren und posterioren Schnittflächen 19, 20.

Die so erhaltenen Koordinaten für die Zielpunkte müssen noch in Ansteuersignale für die dreidimensionale Ablenkeinheit, z.B. die xy-Scanner sowie den z-Scanner umgesetzt werden. Hierzu wird ein entsprechender funktioneller Zusammenhang oder ein entsprechendes Kennfeld verwendet, dass für die Scanner bekannt ist und gegebenenfalls vorab ermittelt wurde.

Insbesondere für die xy-Scanner, die im Ausführungsbeispiel als Galvanometerspiegel realisiert sind, wurde zuvor die Response-Funktion bestimmt. Ein Beaufschlagen der Galvanometerspiegel mit einem Frequenz-Sweep sowie Messen der tatsächlichen Galvanometerbewegung liefern eine Amplituden- und Phasen-Antwortfunktion. Diese werden bei der Bestimmung der Ansteuersignale berücksichtigt.

Weiter wird zur vereinfachten Ansteuerung nicht für jeden Punkt dem Scanner ein Signal für das anzufahrende Ziel vorgegeben. Statt dessen bewirkt das Steuergerät 12 eine Vorgabe von Stützstellen, die die Bahn des Scanners charakterisieren. Die Punktezahl ist dadurch deutlich reduziert. Dies kann schon nutzbringend bei der NL-Transformation ausgenutzt werden, indem nur für die Bahnkurven nur diejenigen Zielpunkte der Transformation unterworfen werden, die Stützstellen bei der Ansteuerung sein sollen. Die Auswertung der Funktionsgleichungen erfolgt in einer Ausführungsform also mittels eines auf einen zeitlichen Abstands der geringer ist, als der Zeitabstand der Laserpulse.

Es wird somit also vor der NL-Transformation eine Filterung der Bahnkurvenpunkte vorgenommen, die die erwähnten Stützstellen, d.h. Punkte in einer Frequenz der Scanneransteuerung zur Transformation vorsieht. Äquivalent mit einer solchen Filterung ist eine Auswertung der funktionsmäßig beschriebenen Bahnkurven an entsprechend der Scannersteuerung beabstandeten Stützstellen. Hier tritt ein weiterer Vorteil des hier geschilderten funktionsbasierten Ansatzes zu Tage: Die Entscheidung, welche Punkte Zielpunkte bei der Ansteuerung der Fokusverstelleinrichtung sind, muss erst vor der NL-Transformation getroffen werden. Zuvor müssen lediglich die Bahnparameter geeignet umgerechnet werden. Auch liegen erst dann Datensätze mit einer Vielzahl an Punkten vor.

Die Stützstellen definieren somit Zielpunkte, die nur eine Teilmenge der Menge der Punkte bilden, an die ein Laserpuls abgegeben wird. Dies ist in Figur 10 veranschaulicht, in der diejenigen Spots 6, die ein Zielpunkt 28 im Steuerdatensatz sind vorliegt, als schwarze ausgefüllte Kreise eingezeichnet sind.

Dieses Vorgehen hat zudem den Vorteil, dass die Maximalfrequenz fs, die bei der Ansteuerung des Scanners auftritt, sehr viel geringer sind, als die Laserpulsfrequenz $f_p$. Beispielsweise kann mit einer Ansteuerfrequenz von 20 kHz sowie einer Laserpulsfrequenz von 200 kHz gearbeitet werden. Im Ergebnis liegen somit zwischen den Zielpunkten 28, die bei der Ansteuerung des Scanners vorgegeben werden, ein oder mehrere Spots 6, auf die ebenfalls gepulste Laserstrahlung abgegeben wird.

Es erfolgt also nicht nur eine Abgabe von gepulster Laserstrahlung, während sich die Scanner in einem Verstellvorgang befinden, beispielsweise während die Galvanometerspiegel sich bewegen, sondern Laserpulse werden von den Scannern abgelenkt, während diese sich von einem vorgegebenen Zielpunkt zum nächsten bewegen. Um eine möglichst hohe Ablenkgeschwindigkeit zu erreichen, stellt diese Bewegung eine Schwingung dar, die bei perfekten Kreisspiralen (wie sie bei der anterioren Schnittfläche 19 auftreten) sogar eine rein sinusförmige Schwingung ist. Da auch bei der Lentikel-Fläche 20 die tatsächliche Spiralform nur wenig von idealen Kreis- oder elliptischen Spiralen abweicht, können die Scanner nahe ihrer Grenzfrequenz betrieben werden, so dass die beschriebenen Bahnen, entlang derer die Spots angeordnet sind, eine sehr schnelle Schnittflächenerzeugung erlauben.

Nach der Ermittlung der Steuerdatensätze enthaltend der Zielpunkte aus den geschilderten Punktmengen, die für die Bahnkurven erhalten wurden, ist das Vorverfahren abgeschlossen, das zur Bereitstellung der entsprechenden Steuerwert oder -parameter durchgeführt wurde. Für dieses Vorverfahren ist eine menschliche Mitwirkung und insbesondere die Mitwirkung eines Arztes oder Chirurgen nicht erforderlich. Das Verfahren wird vom Steuergerät 12 ohne Tätigkeit eines Mediziners ausgeführt. Dessen Anwesenheit ist erst für den nachgelagerten chirurgischen Eingriff erforderlich.

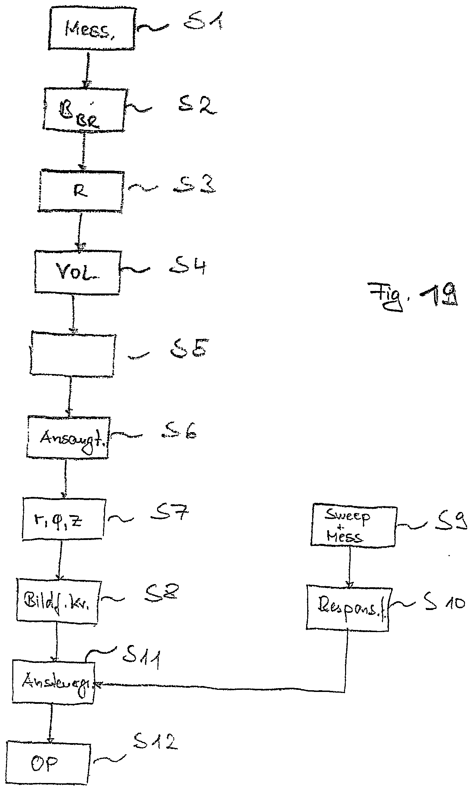

Der Ablauf des Verfahrens zur Vorbereitung des Gerätes 1 auf den Einsatz bei einer augenchirurgischen Fehlsichtigkeitsoperation ist schematisch in Figur 19 zusammengefasst. In einem Schritt S1 erfolgt die Vermessung des Auges 3. Dabei werden für die beim Patienten 4 vorliegenden Fehlsichtigkeit Korrekturparameter erhalten, wie sie z.B. für herkömmliche Brillen üblich sind. Die in Schritt S2 aufgestellten Parameter werden dann in einem Schritt S3 dazu verwendet, die zur Korrektur nötige neue Krümmung der Hornhaut 5 zu bestimmen. Ist diese Berechnung in Schritt S3 abgeschlossen, wird das Volumen in S4 bestimmt, das aus der Hornhaut entnommen werden muss. Dies geschieht üblicherweise unter Bestimmung der Lentikel-Fläche 20 sowie der Flap-Fläche 19 in einem Schritt S5. Hat man die entsprechenden Funktionsbeschreibungen dieser Flächen gewonnen, wird in Schritt S6 die Ansaugtransformation, die sich beim Ansaugen des Auges an das Kontaktglas auswirkt, berücksichtigt.

Als nächstes werden die Koordinaten der Bahnkurven ermittelt, aus denen die Schnittflächen aufgebaut werden. Dies ist schematisch in Schritt S7 durch die Parameter r, $\varphi$, z angedeutet. Am Ende des Schrittes S7 liegt ein Punkt-Muster mit den Koordinaten der Spots, auf die ein Laserstrahlungspuls einwirken soll. Die Dichte der Zielpunkte kann dabei bereits zur Vereinfachung des rechnerischen Aufwandes reduziert sein, indem nicht für jeden mit Laserstrahlung beaufschlagten Spot eine Stützstelle bei der Ansteuerung der Scanner angegeben wird.

Nachfolgend wird die derart erhaltene Koordinatenmenge in Schritt S8 zur Berücksichtigung des Fokuslagenfehlers nochmals transformiert. In einem Schritt S11 werden dann die eigentlichen Ansteuerparameter ermittelt, wobei eine Response-Funktion eingeht, in einem Schritt S10 aus einer vorherigen Messung (Schritt S9) des Amplituden- und Frequenzverhaltens der Scanner erhalten wurde.

Mit den derart ermittelten Ansteuerparametern wird dann in Schritt S12 die eigentliche Operation durchgeführt, bei der nun vorzugsweise zwischen den einzelnen Stützstellen, die bei der Ansteuerung des Scanners zugrunde liegen, zusätzliche Spots mit Laserstrahlungspulsen beaufschlagt werden.

**Patentansprüche**

1. Behandlungsvorrichtung zur operativen Fehlsichtigkeitskorrektur eines Auges (3) eines Patienten (4), die eine Lasereinrichtung (L), welche durch Einstrahlen gepulster fokussierter Laserstrahlung (2) Hornhaut-Gewebe trennt, ein Kontaktglas (25) zum Aufsetzen und Fixieren des Auge (3), das der Hornhautvorderfläche (15) eine bekannte sphärische Form verleiht, und eine Planungseinrichtung (P) aufweist, wobei die Planungseinrichtung (P)

   - eine Schnittstelle (S) zum Zuführen von Messdaten über Parameter des Auges (3) und Fehlsichtigkeitsdaten über die zu korrigierenden Fehlsichtigkeit des Auges (3) aufweist,
   - aus zugeführten Mess- und Fehlsichtigkeitsdaten ein Volumen (18) definiert, das innerhalb der Hornhaut (5) liegt und dessen Entfernung aus der Hornhaut (5) die gewünschte Fehlsichtigkeitskorrektur bewirkt,
   - eine Grenzfläche (19, 20) festlegt, die das definierte Volumen (18) innerhalb der Hornhaut (5) begrenzt, und
   - für diese Grenzfläche (19, 20) einen Steuerdatensatz zur Ansteuerung der Lasereinrichtung (L) erzeugt, der in der Hornhaut (5) ein dreidimensionales Muster an Zielpunkten (28) festlegt, die in der Grenzfläche (19, 20) liegen und so angeordnet sind, dass die Grenzfläche (19, 20) nach Einstrahlung der gepulsten Laserstrahlung (2) gemäß dem Steuerdatensatz als Schnittfläche ausgebildet ist, die das definierte Volumen (18) in der Hornhaut (5) isoliert und so entfernbar macht,
   **dadurch gekennzeichnet, dass**
   - die Planungseinrichtung (P) die Grenzfläche aus einer anterioren Teilfläche (19) und einer posterioren Teilfläche (20) zusammensetzt, wobei die anteriore Teilfläche (19) ein Paraboloid ist, das eine Sphäre, die in einem konstanten Abstand zur Hornhautvorderfläche (15) liegt, approximiert, und die posteriore Teilflächen (20) keinen konstanten Abstand zur Hornhautvorderfläche (15) hat.

2. Behandlungsvorrichtung nach Anspruch 1, **dadurch gekennzeichnet, dass** die Fehlsichtigkeitsdaten die Brechkraft BBR einer für die Fehlsichtigkeitskorrektur tauglichen Brille (17), sowie den Abstand dHS umfassen, in dem die Brille (17) mit der Brechkraft BBR vor dem Hornhautscheitel liegen muss, um die gewünschte Fehlsichtigkeitskorrektur mittels der Brille (17) zu erreichen.

3. Behandlungsvorrichtung nach Anspruch 2, **dadurch gekennzeichnet, dass** die Planungseinrichtung (P) das Volumen (18) so definiert, dass Hornhautvorderfläche (15*) des Auges (3) nach Entfernung des Volumens (18) einen Krümmungsradius RCV* annimmt, und dabei folgende Gleichung verwendet

$$RCV^* = 1 / (\,(1/RCV) + BBR / (\,(nc-1)\,(1 - dHS \cdot BBR))) + F,$$

   wobei RCV der Krümmungsradius der Hornhaut (5) vor Entfernung des Volumens (18), nc die Brechkraft des Materials der Hornhaut (5) und F ein Faktor ist, wobei insbesondere

$$F = (1 - 1/nc) \cdot (dC^* - dC)$$

   gilt, wobei dC bzw. dC* die Dicke der Hornhaut (5, 5*) bzw. nach Entfernung des Volumens (18) bezeichnet und das Planungsmodul (P) den Radius RCV* iterativ berechnet, indem bei jedem Iterationsschritt aus der Differenz (RCV* - RCV) auf die Größe (dC* - dC) geschlossen wird und das entsprechende daraus erhaltene Ergebnis für die Dickenänderung bei der Berechnung von RCV* im nächsten Iterationsschritt angewendet wird.

4. Behandlungsvorrichtung nach einem der obigen Ansprüche, **dadurch gekennzeichnet, dass** die Lasereinrichtung (L) eine dreidimensionale Fokusverstelleinrichtung (9,11) aufweist, die die fokussierte Laserstrahlung (2) entlang einer Bahn (24) über das Muster der Zielpunkte (28) verstellt und Pulse der gepulsten Laserstrahlung (2) in die Hornhaut (5) auch auf Punkte (6) abgibt, die auf der Bahn (24) zwischen den Zielpunkten (28) liegen.

5. Behandlungsvorrichtung nach einem der obigen Ansprüche, **dadurch gekennzeichnet, dass** die Lasereinrichtung (L) eine dreidimensionale Fokusverstelleinrichtung (9,11) aufweist und das Steuergerät (12) konfiguriert ist, die dreidimensionale Fokusverstelleinrichtung (9, 11) so anzusteuern, dass diese den Fokus (7) der fokussierten Laserstrahlung (2) entlang einer Bahn verstellt, wobei die Behandlungsvorrichtung die gepulste Laserstrahlung bei fortlaufender Verstellung der Fokuslage appliziert und zur Steuerung der Lage des Fokus (7) zylindrische oder Kugel-Koordinaten verwendet sind.

6. Behandlungsvorrichtung nach einem der obigen Ansprüche, **dadurch gekennzeichnet, dass** die Grenzfläche (19, 20) ein Volumen (18) innerhalb der Hornhaut (5) begrenzt, in der Hornhaut (5) isoliert und so entfernbar macht und das Volumen (18) die Pupille des dunkelangepassten Auges (3) überdeckt.

7. Behandlungsvorrichtung nach einem der obigen Ansprüche, **dadurch gekennzeichnet, dass** eine Datenverbindung oder ein Datenträger zum Übertragen der Steuerdaten von der Planungseinrichtung (P) an die Lasereinrichtung (L) vorgesehen ist.

8. Behandlungsvorrichtung nach einem der obigen Ansprüche, **dadurch gekennzeichnet, dass** eine Messeinrichtung (M) zum Ermitteln von Messdaten über Parameter des Auges (3) und Fehlsichtigkeitsdaten über die zu korrigierenden Fehlsichtigkeit des Auges (3) direkt mit der Behandlungsvorrichtung (1) verbunden ist.

9. Behandlungsvorrichtung nach einem der obigen Ansprüche, **dadurch gekennzeichnet, dass** sie eine Anzeigeeinrichtung zur visuellen Darstellung der Steuerdaten und eine Eingabeeinrichtung zum nachträglichen Verändern der Steuerdaten aufweist.

10. Behandlungsvorrichtung nach einem der obigen Ansprüche, **dadurch gekennzeichnet, dass** die Lasereinrichtung (L) eine dreidimensionale Fokusverstelleinrichtung (9,11) aufweist und das Steuergerät (12) konfiguriert ist, die dreidimensionale Fokusverstelleinrichtung (9, 11) so anzusteuern, dass diese den Fokus (7) der fokussierten Laserstrahlung (2) entlang einer Bahn verstellt, welche eine Kurve in Form einer Spirale aufweist.

11. Behandlungsvorrichtung nach einem der obigen Ansprüche, **dadurch gekennzeichnet, dass** die Hornhautverformung, die bei der Anwendung des gekrümmten Kontaktglases (25) auftritt, im Steuerdatensatz berücksichtigt sind, damit unabhängig vom Grad der Verformung eine gewünschte Grenzfläche (19, 20) im nicht durch das Kontaktglas (25) verformte Auge (3) vorliegt.

12. Behandlungsvorrichtung nach einem der obigen Ansprüche, **dadurch gekennzeichnet, dass** die Lasereinrichtung (L) mittels einer Optik die Laserstrahlung (2) in einen Fokus (7), der einen Spot (6) überdeckt, fokussiert und Pulse der Laserstrahlung (2) in einen Bereich der Hornhaut (5) abgibt, wobei sich für mehrere der Pulse die Spots (6) überlappen.

## Claims

1. A treatment apparatus for the surgical correction of defective vision in an eye (3) of a patient (4), which has a laser device (L) that separates corneal tissue by irradiating pulsed, focused laser radiation (2) , a contact glass (25) for placing on and fixing the eye (3), which gives the corneal front surface (15) a known spherical shape, and a planning device (P), wherein the planning device (P)

- comprises an interface (S) for receiving measurement data about parameters of the eye (3) and defective vision data about the defective vision of the eye (3) to be corrected,
- defines a volume (18) from the received measurement and defective vision data, which volume is situated within the cornea (5) and the removal of which from the cornea (5) effects the desired defective vision correction,
- defines a boundary surface (19, 20) which confines the defined volume (18) within the cornea (5), and
- for this boundary surface (19, 20) generates a control data set for controlling the laser device (L), which defines in the cornea (5) a three-dimensional pattern of target points (28) which lie in the boundary surface (19, 20) and are arranged in such a way that, after irradiating the pulsed laser radiation (2) in accordance with the control data set, the boundary surface (19, 20) is formed as a cut surface which isolates the defined volume (18) in the cornea (5) and thus makes it removable,
**characterized in that**
- the planning device (P) composes the boundary surface from an anterior surface part (19) and a posterior surface part (20), the anterior surface part (19) being a paraboloid that approximates a sphere that lies at a constant distance from the corneal front surface (15), and the posterior partial surface (20) does not have a constant distance from the corneal front surface (15).

2. The treatment apparatus according to claim 1, **characterized in that** the defective vision data includes the refractive power BBR of a pair of glasses (17) suitable for correcting defective vision, and the distance dHS, at which the spectacles (17) with the refractive power BBR must lie in front of the apex of the cornea in order to achieve the desired correction of defective vision by means of the spectacles (17).

3. The treatment apparatus according to claim 2, **characterized in that** the planning device (P) defines the volume (18) such that the corneal front surface (15*) of the eye (3) assumes a radius of curvature RCV* after removal of the volume (18), and uses the following equation for this purpose

$$RCV^* = 1 / ( (1/RCV) + BBR / ( (nc\text{-}1) (1 - dHS \cdot BBR))) + F,$$

where RCV is the radius of curvature of the cornea (5) before removal of the volume (18), nc is the refractive power of the material of the cornea (5) and F is a factor, in particular

$$F = (1 - 1/nc) \cdot (dC^* - dC),$$

where dC and dC* denote the thickness of the cornea (5, 5*) and after removal of the volume (18), respectively, and the planning module (P) iteratively calculates the radius RCV* by iteration step, the difference (RCV* - RCV) is used to infer the size (dC* - dC) and the corresponding result obtained therefrom for the change in thickness is used in the calculation of RCV* in the next iteration step.

4. The treatment apparatus according to one of the above claims, **characterized in that** the laser device (L) comprises a three-dimensional focus adjustment device (28) which adjusts the focused laser radiation (2) along a path (24) over the pattern of target points (28) and also emitting pulses of the pulsed laser radiation (2) into the cornea (5) at points (6) which lie on the path (24) between the target points (28).

5. The treatment apparatus according to one of the above claims, **characterized in that** it comprises a three-dimensional focus adjustment device and the control unit (12) is configured to control the three-dimensional focus adjustment device (9, 11) so as to that it adjusts the three-dimensional focus adjustment device (9, 11) in such a way that the latter adjusts the focus (7) of the focused laser radiation (2) along a path, the treatment apparatus applying the pulsed laser radiation during continuous adjustment of the focus position, and cylindrical or spherical coordinates being used to control the position of the focus (7).

6. The treatment apparatus according to one of the above claims, **characterized in that** the boundary surface (19, 20) bounds a volume (18) within the cornea (5), isolates it in the cornea (5) and thus makes it removable, and the volume (18) covers the pupil of the dark-adapted eye (3).

7. The treatment apparatus according to one of the above claims, **characterized in that** a data link or a data carrier is provided for transmitting the control data from the planning device (P) to the laser device (L).

8. The treatment apparatus according to one of the above claims, **characterized in that** a measuring device (M) for determining measurement data relating to parameters of the eye (3) and defective vision data relating to the defective vision of the eye (3) to be corrected is connected directly to the treatment apparatus (1).

9. The treatment apparatus according to one of the above claims, **characterized in that** it comprises a display device for visually displaying the control data and an input device for subsequently modifying the control data.

10. The treatment apparatus according to one of the above claims, **characterized in that** it comprises a three-dimensional focus adjustment device and the control unit (12) is configured to control the three-dimensional focus adjustment device (9, 11) so that it adjusts the focus (7) of the focused laser radiation (2) along a path which comprises a curve in the form of a spiral.

11. The treatment apparatus according to one of the above claims, **characterized in that** the corneal deformation that occurs when the curved contact glass (25) is used is taken into account in the control data set, so that a desired interface (19, 20) is present in the eye (3) not deformed by the contact glass (25) independently of the degree of deformation.

12. The treatment apparatus according to one of the above claims, **characterized in that** the laser device (L) focuses the laser radiation (2) by means of an optical system into a focus (7) which covers a spot (6), and emits pulses of the laser radiation (2) into an area of the cornea (5), the spots (6) overlapping for a plurality of the pulses.

**Revendications**

1. Appareil de traitement pour la correction chirurgicale d'un défaut de vision d'un oeil (3) d'un patient (4), qui possède un dispositif laser (L) qui sépare des tissus cornéens par irradiation d'un rayonnement laser focalisé pulsé (2), un verre de contact (25) pour la mise en place et la fixation de l'oeil (3), qui confère une forme sphérique connue à la face antérieure de la cornée (15), et un dispositif de planification (P), le dispositif de planification (P)

   - possède une interface (S) pour l'apport de données de mesure concernant des paramètres de l'œil (3) et de données de défauts visuels concernant les défauts visuels de l'œil (3) à corriger,
   - définit, à partir des données de mesure et d'amétropie fournies, un volume (18) qui se trouve à l'intérieur de la cornée (5) et dont l'enlèvement de la cornée (5) provoque la correction souhaitée de l'amétropie,
   - détermine une surface limite (19, 20) qui délimite le volume défini (18) à l'intérieur de la cornée (5), et
   - pour cette surface limite (19, 20), on génère un jeu de données de commande pour commander le dispositif laser (L), qui définit dans la cornée (5) un modèle tridimensionnel de points cibles (28) qui se trouvent dans la surface limite (19, 20) et qui sont disposés de telle sorte que la surface limite (19, 20), après irradiation du rayonnement laser pulsé (2) selon le jeu de données de commande, est réalisée sous forme de surface de coupe qui isole le volume défini (18) dans la cornée (5) et le rend ainsi éliminable,
   **caractérisé en ce que**
   - le dispositif de planification (P) compose l'interface d'une surface partielle antérieure (19) et d'une surface partielle postérieure (20), la surface partielle antérieure (19) étant un paraboloïde approximant une sphère située à une distance constante de la surface antérieure de la cornée (15), et la surface partielle postérieure (20) n'étant pas à une distance constante de la surface antérieure de la cornée (15).

2. Appareil de traitement selon la revendication 1, **caractérisé en ce que** les données relatives à l'amétropie comprennent la puissance de réfraction BBR d'une paire de lunettes (17) adaptée à la correction de l'amétropie, ainsi que la distance dHS à laquelle la paire de lunettes (17) avec la puissance de réfraction BBR doit se trouver devant le sommet de la cornée pour obtenir la correction souhaitée de l'amétropie au moyen de la paire de lunettes (17).

3. Appareil de traitement selon la revendication 2, **caractérisé en ce que** le dispositif de planification (P) définit le volume (18) de telle sorte que la surface antérieure de la cornée (15*) de l'oeil (3) prenne un rayon de courbure RCV* après retrait du volume (18), en utilisant l'équation suivante

$$RCV^* = 1 / ( (1/RCV) + BBR / ( (nc-1) (1 - dHS - BBR)) + F,$$

   où RCV est le rayon de courbure de la cornée (5) avant l'enlèvement du volume (18), nc est le pouvoir réfringent du matériau de la cornée (5) et F est un facteur, avec en particulier

$$F = (1 - 1/nc) - (dC^* - dC)$$

   où dC ou dC* désigne l'épaisseur de la cornée (5, 5*) ou après enlèvement du volume (18), et le module de planification (P) calcule le rayon RCV* de manière itérative en déduisant à chaque étape d'itération la grandeur (dC* - dC) à partir de la différence (RCV* - RCV) et en appliquant le résultat correspondant obtenu pour la variation d'épaisseur lors du calcul de RCV* à l'étape d'itération suivante.

4. Appareil de traitement selon l'une des revendications précédentes, **caractérisé en ce que** le dispositif laser (L) possède un dispositif de réglage de focalisation tridimensionnel (28) qui déplace le rayonnement laser focalisé (2) le long d'une trajectoire (24) sur le modèle des points cibles (28) et délivre des impulsions du rayonnement laser pulsé (2) dans la cornée (5) également sur des points (6) qui se trouvent sur la trajectoire (24) entre les points cibles (28).

5. Appareil de traitement selon l'une des revendications précédentes, **caractérisé en ce qu'**il possède un dispositif de réglage du foyer tridimensionnel et **en ce que** l'appareil de commande (12) est configuré pour commander le dispositif de réglage du foyer tridimensionnel (9, 11) de manière à déplacer le foyer (7) du rayonnement laser focalisé (2) le long d'une trajectoire, l'appareil de traitement appliquant le rayonnement laser pulsé avec un réglage continu de la position du foyer et des coordonnées cylindriques ou sphériques étant utilisées pour commander la position du foyer (7).

6. Appareil de traitement selon l'une des revendications précédentes, **caractérisé en ce que** l'interface (19, 20) délimite un volume (18) à l'intérieur de la cornée (5), l'isole dans la cornée (5) et le rend ainsi amovible, et le volume (18) recouvre la pupille de l'œil (3) adapté à l'obscurité.

7. Appareil de traitement selon l'une des revendications précédentes, **caractérisé en ce qu'**il est prévu une liaison de données ou un support de données pour transmettre les données de commande du dispositif de planification (P) au dispositif laser (L).

8. Appareil de traitement selon l'une des revendications précédentes, **caractérisé en ce qu'**un dispositif de mesure (M) destiné à déterminer des données de mesure concernant des paramètres de l'œil (3) et des données de défaut de vision concernant le défaut de vision de l'œil (3) à corriger est directement relié à l'appareil de traitement (1).

9. Appareil de traitement selon l'une des revendications précédentes, **caractérisé en ce qu'**il possède un dispositif d'affichage pour représenter visuellement les données de commande et un dispositif d'entrée pour modifier ultérieurement les données de commande.

10. Appareil de traitement selon l'une quelconque des revendications précédentes, **caractérisé en ce qu'**il possède un dispositif de réglage de foyer tridimensionnel et **en ce que** l'appareil de commande (12) est configuré pour commander le dispositif de réglage de foyer tridimensionnel (9, 11) de manière à déplacer le foyer (7) du rayonnement laser focalisé (2) le long d'une trajectoire possédant une courbe en forme de spirale.

11. Appareil de traitement selon l'une des revendications précédentes, **caractérisé en ce que** la déformation de la cornée qui se produit lors de l'utilisation du verre de contact curviligne (25) est prise en compte dans l'enregistrement de commande, afin qu'une interface souhaitée (19, 20) soit présente dans l'œil (3) non déformé par le verre de contact (25), indépendamment du degré de déformation.

12. Appareil de traitement selon l'une des revendications précédentes, **caractérisé en ce que** le dispositif laser (L) focalise, au moyen d'une optique, le rayonnement laser (2) en un foyer (7) qui recouvre un spot (6), et émet des impulsions du rayonnement laser (2) dans une zone de la cornée (5), les spots (6) se chevauchant pour plusieurs des impulsions.

## FIG 1

## FIG 2

Fig. 5

Fig. 6

Fig. 1a

FIG 3

Fig. 7

Fig. 4

a)

b)

c)

FIG 8

FIG 9

Fig 10

Fig. 11

FIG 12

Fig. 13

Fig. 14

Fig. 15

Fig. 18

Fig. 16

Fig. 17

Fig. 19

**EP 4 342 436 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1719483 A1
- US 5984916 A
- WO 2005011545 A1
- EP 1159986 A1
- US 5549632 A
- DE 69500997 T2
- WO 2004032810 A2
- WO 2005011546 A
- WO 2005011545 A
- WO 2005048895 A
- WO 2003002008 A
- WO 2005011547 A1